# EUROPÄISCHE PATENTSCHRIFT

(11) **EP 1 070 082 B1**
(45) Veröffentlichungstag und Bekanntmachung des Hinweises auf die Patenterteilung: **17.09.2003**
(21) Anmeldenummer: 99924752.1
(22) Anmeldetag: 26.03.1999
(51) Int. Cl.: C07J 9/00, C07J 7/00, C07J 51/00

(54) **VERFAHREN ZUR HERSTELLUNG VON 4,4-DIMETHYL-5 ALPHA-CHOLESTA-8,14,24-TRIEN-3 BETA-OL UND ZWISCHENPRODUKTE IM VERFAHREN (I)**
METHOD FOR PRODUCING 4,4-DIMETHYL-5ALPHA-CHOLESTA-8,14,24-TRIENE-3BETA-OL AND INTERMEDIATE PRODUCTS OBTAINED BY SAID METHOD
PROCEDE DE PREPARATION DE 4,4-DIMETHYL-5ALPHA-CHOLESTA-8,14,24-TRIEN-3BETA-OL ET DE PRODUITS INTERMEDIAIRES OBTENUS SELON LEDIT PROCEDE

(30) Priorität: 09.04.1998 DE 19817520; 20.05.1998 DE 19823677
(43) Veröffentlichungstag der Anmeldung: 24.01.2001
(73) Patentinhaber: Schering Aktiengesellschaft, 13353 Berlin (DE)
(72) Erfinder: BLUME, Thorsten, D-13467 Berlin (DE); ESPERLING, Peter, D-12107 Berlin (DE); KUHNKE, Joachim, 14469 Potsdam (DE)
(86) Internationale Anmeldenummer: DE9901002
(87) Internationale Veröffentlichungsnummer: WO99052930

(56) Entgegenhaltungen:
- RUAN B ET AL: "An alternative synthesis of 4,4-Dimethyl-5alpha-cholesta-8,14,24 -trien-3beta-ol, an intermediate in sterol biosynthesis and a reported activator of meiosis and of nuclear orphan receptor LXRalpha" BIOORGANIC & MEDICINAL CHEMISTRY LETTERS, Bd. 8, Nr. 3, 3. Februar 1998 (1998-02-03), Seite 233-236 XP004136854 ISSN: 0960-894X in der Anmeldung erwähnt
- DOLLE R E ET AL: "SYNTHESIS OF ZYMOSTEROL, FECOSTEROL, AND RELATED BIOSYNTHETIC STEROL INTERMEDIATES 1,2" JOURNAL OF THE AMERICAN CHEMICAL SOCIETY, Bd. 111, Nr. 1, 4. Januar 1989 (1989-01-04), Seiten 278-284, XP000611665 ISSN: 0002-7863 in der Anmeldung erwähnt
- LETTRE, HANS ET AL: "Polyols derived from sterols and sterol derivatives. VI. Steroids containing structural units of ecdysone and the elatericins" LIEBIGS ANNALEN DER CHEMIE, Bd. 758, 1972, Seiten 89-110, XP002111484 WEINHEIM DE
- R. B. WOODWARD ET AL: "The Synthesis of Lanosterol (Lanostadienol)" JOURNAL OF THE CHEMICAL SOCIETY., Nr. 3, März 1957 (1957-03), Seiten 1131-1144, XP002111485 CHEMICAL SOCIETY. LETCHWORTH., GB
- R. E. DOLLE ET AL: "Improved Preparation of (3.beta.,5.alpha.,14.alpha)-3-Hydroxy-14-m ethylcholest-7-en-15-one. Synthesis of Ergostenone and 20.alpha.-(Hydroxymethyl)pregnenone Analogues" JOURNAL OF ORGANIC CHEMISTRY., Bd. 51, Nr. 21, 17. Oktober 1986 (1986-10-17), Seiten 4047-4053, XP002111486 AMERICAN CHEMICAL SOCIETY. EASTON., US ISSN: 0022-3263
- J. VAN DER EYCKEN ET AL: "24(R),25-Dihydroxycholesterol; An Attempt for Side Chain Stereocontrol via Iodolactonization" BULLETIN DES SOCIETES CHIMIQUES BELGES, Bd. 95, Nr. 4, 1986, Seiten 289-292, XP002111487

## Beschreibung

Die Erfindung betrifft Verfahren zur Herstellung von 4,4-Dimethyl-5α-cholesta-8,14,24-trien-3β-ol (**1**) und Zwischenprodukte im Verfahren 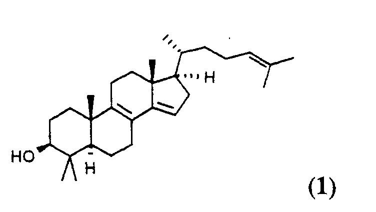 Untersuchungen von Byskov et. al. (*Nature* **1995,** *374*, 559) zeigen, daß 4,4-Dimethyl-5α-cholesta-8,14,24-trien-3β-ol, Formel **1**, im folgenden FF-MAS genannt, isoliert aus menschlicher Follikelflüssigkeit, eine die Meiose regulierende endogene Substanz ist, der interessante hormonelle Effekte zugeschrieben werden. Somit ist diese Substanz für pharmazeutische Anwendungen, beispielsweise zur Fertilitätsförderung, von Bedeutung.

Eine erste Synthese dieses Naturstoffes, welcher in der Biosynthese von Cholesterin aus Lanosterol durchlaufen wird, wurde von Dolle et al. (*J. Am. Chem. Soc*. **1989,** *111*, 278) beschrieben. Ausgehend von Ergosterin wird FF-MAS in einer 18stufigen aufwendigen Synthesesequenz erhalten. Große Teile der Synthese sind dem chemischen Teilabbau der Ergosterinseitenkette, dem anschließenden Aufbau der FF-MAS Seitenkette und der zur Erreichung dieses Ziels notwendigen Schutzgruppenchemie gewidmet.

Eine zweite Synthese von FF-MAS wurde von Schroepfer et al. ausgehend von Dehydrocholesterol in einer 13stufigen Synthese beschrieben (Bioorg. Med. Chem. Lett. **1997,** 8, 233). Auch in dieser Synthese muß zum Seitenkettenaufbau ein aufwendiger Schutz des Dien-Systems durchgeführt werden. Allein vier Schritte (Epoxydierung und Umlagerung zum Schutz; Reduktion und Eliminierung zur Regeneration des Dien-Systems) sind der Schutzgruppenstrategie geschuldet.

Aufgabe dieser Erfindung sind neue Verfahren zur Synthese von FF-MAS. Ebenfalls Gegenstand dieser Erfindung sind die neuen, bisher nicht bekannten Verbindungen, die im Rahmen der Synthesen durchlaufen werden und per se oder derivatisiert als Ausgangsmaterialien für die Synthese anderer Zielmoleküle verwendet werden können, beispielsweise zur Synthese von FF-MAS Analoga (siehe WO 96/00235) und die Verwendung von Verbindungen zur Herstellung von 4,4-Dimethyl-5α-cholesta-8,14,24-trien-3β-ol.

### Gelöst wird diese Aufgabe durch die Lehre der Patentansprüche

Durch die beiden erfindungsgemäßen Verfahren werden deutlich weniger Zwischenstufen durchlaufen als in der bekannten Synthese von Dolle et. al. Die Anzahl von Reinigungsschritten ist deutlich niedriger, und es sind keine technisch aufwendigen Einrichtungen, wie ein Ozongenerator mit den zu seinem Betrieb notwendigen Installationen, nötig.

### Verfahrensvariante 1:

Gemäß Schema 1 wird FF-MAS ausgehend beispielsweise von 3-Oxopregn-4-en-21-säuremethylester (Formel **2** mit R¹ = CH₃) (Helv. Chim. Acta **1939,** 22, 1178 und 1184) in einer 10stufigen Sequenz hergestellt. Die hier als Edukt genannte Verbindung ist auf verschiedenen Wegen leicht aus kommerziell erhältlichen Steroiden zugänglich. Beispielsweise ist die Darstellung einer Verbindung der Formel **2** mit R¹ = CH₃ in einer 3stufigen Sequenz aus 3β-Hydroxyandrost-5-en-17-on (CAS Registry Nummer 53-43-0; 571-35-7 etc.) via Horner-Wittig (z.B. *Synth. Commun.* **1977,** *7,* 215), Reduktion der resultierenden 17-Doppelbindung (z.B. *Synthesis* **1996,** 455) und anschließender Oppenauer-Oxidation (z.B. *Helv. Chim. Acta* **1939,** 22, 1178 und 1184) beschrieben.
Ausgehend von 3β-Acetoxy-androst-5-en-17-on (CAS Registry Nummer 853-23-6 etc.) ist eine Verbindung der Formel **2,** mit R¹ = CH₃ auch via Kondensation mit Malodinitril, anschließender Reduktion der resultierenden 17,20-Doppelbindung mit Natriumborhydrid, Nitrilverseifung und Decarboxylierung mit Kaliumhydroxid in Ethylenglykol, Veresterung der resultierenden Carbonsäure (*Coll. Czech. Chem. Commun*. **1982,** 1240) und abschließender Oppenauer-Oxidation (z.B. *Helv. Chim. Acta* **1939,** *22*, 1178 und 1184) darstellbar.

Dem Fachmann ist geläufig, daß R¹ in Verbindungen der Formel **2** nach Standardmethoden variiert werden kann. Dies kann durch Verwendung anderer Alkohole im Veresterungsschritt geschehen, aber auch durch Umesterung eines schon vorhandenen Esters. R¹ kann also die Bedeutung von Wasserstoff, Methyl, Ethyl, Propyl, Isopropyl, Butyl und die entsprechenden Butylisomere, Pentyl und die entsprechenden Pentylisomere sowie Hexyl und die entsprechenden Hexylisomere, Phenyl, Benzyl, ortho-, meta- und para-Methylphenyl besitzen.

Die Umsetzung einer Verbindung der Formel 2 zu einer Verbindung der Formel **3** erfolgt nach an sich bekannten Verfahren (z.B. *Helv. Chim. Acta* **1980,** *63,* 1554; *J. Am. Chem. Soc*. **1954,** *76,* 2852). Beispielsweise wird eine Verbindung der Formel 2 in Gegenwart von Basen wie etwa den Alkalisalzen niederer Alkohole, bevorzugt aber Kaliumtertiärbutylat, mit einem Alkylierungsmittel wie etwa Dimethylsulfat, Dimethylcarbonat oder auch Methyliodid in einem Lösemittel oder Lösemittelgemisch umgesetzt. Als Lösemittel können niedere, bevorzugt tertiäre Alkohole,sowie Ether, beispielsweise Methyltertiärbutylether oder Tetrahydrofuran und deren Gemische,verwendet werden. Bevorzugt ist die Verwendung von tertiär-Butanol bzw. eines Gemisches aus tertiär-Butanol und Tetrahydrofuran. Die Reaktion wird in einem Temperaturbereich von 0°C bis 65°C durchgeführt, bevorzugt aber im Temperaturbereich von 15°C bis 50°C.

Die Umsetzung eines Ketones der Formel **3** in den entsprechenden 3-Alkohol der Formel **4** läßt sich mit einer Vielzahl von Reduktionsmitteln durchführen. Als Beispiele seien genannt: BH₃-Komplexe (z.B. mit tertiär-Butylamin oder Trimethylamin), Selectride, Natrium- und Lithiumborhydrid, gebremste Lithiumluminiumhydride (z.B. LiAl(O^{t}Bu)₃H); auch sind Mikroorganismen wie z.B. Bäckerhefen oder Enzyme, beispielsweise 3β-Hydroxysteroiddehydrogenase, verwendbar.

Dem Fachmann ist bekannt, daß je nach verwendetem Reagenz verschiedene Lösemittel oder Lösemittelgemische und Reaktionstemperaturen zum Einsatz kommen. Bevorzugt werden hier jedoch Borhydride, wie etwa Natriumborhydrid in geeigneten Lösemitteln wie etwa niederen Alkoholen oder Gemische von Alkoholen mit aprotischen Lösemitteln, beispielsweise Dichlormethan oder Tetrahydrofuran. Die Umsetzungen werden in einem Temperaturbereich von -20°C bis 40°C, bevorzugt jedoch im Bereich von 0°C bis 30°C durchgeführt.

Vor der Einführung der 7,8-Doppelbindung (**5→6**) wird die 3-OH Gruppe einer Verbindung der Formel **4** mit einer für diese Umsetzung geeigneten Schutzgruppe R² versehen. Als Schutzgruppen eignen sich beispielsweise Ester aliphatischer und aromatischer Carbonsäuren, z.B. Essig- und Benzoesäureester, Acetalschutzgruppen wie etwa Tetrahydropyranyl-, Methoxymethyl- oder Methoxyethoxymethylether, aber auch andere Etherschutzgruppen, beispielsweise Silylether wie etwa Trimethylsilyl-, Triethylsilyl- oder Triisopropylsilyl; Triphenylsilyl; Dimethyl(1,1-dimethylethyl)silylether.

Je nach gewünschter Schutzgruppe variieren die Reaktionsbedingungen und Reaktionstemperaturen. Die Einführung der jeweiligen Schutzgruppe erfolgt nach an sich dem Fachmann bekannten Verfahren. Als Beispiel sei die Veresterung einer Verbindung der Formel **4** mit Acetylchlorid in Gegenwart einer Base wie Triethylamin oder Pyridin mit oder auch ohne Zusatz eines inerten Lösemittels, beispielsweise Dichlormethan im Temperaturbereich von 0°C bis 60°C, genannt. Die Einführung einer Silylschutzgruppe erfolgt bevorzugt durch Umsetzung einer Verbindung der Formel **4** mit einem Silylhalogenid, bevorzugt jedoch Dimethyl-(1,1-dimethylethyl)-silyl-chlorid oder Triethylsilylchlorid in Gegenwart einer Base, beispielsweise Imidazol, in einem geeigneten Lösemittel wie etwa Dimethylformamid in einem Temperaturbereich von 10°C bis 140°C, bevorzugt jedoch zwischen 20°C und 100°C. Die Einführung der 7,8-Doppelbindung in eine Verbindung der Formel **5** (→**6**) kann in einem 2stufigen Verfahren erfolgen. Zunächst wird allylisch zur 5,6-Doppelbindung in Position 7 bromiert und anschließend wird durch Eliminierung von Bromwasserstoff eine Verbindung der Formel **6** erhalten. Die Bromverbindung braucht nicht isoliert zu werden, sondern kann in der Regel sofort in die nächste Stufe eingesetzt werden. Die Bromierung erfolgt nach an sich bekannten Verfahren. Beispielsweise kann N-Bromsuccinimid in einem geeigneten Lösemittel, wie etwa Benzol, niederen Alkanen oder auch halogenierten Kohlenwasserstoffen wie etwa Tetrachlorkohlenstoff verwendet werden. Die Reaktion kann unter Zusatz eines Radikalstarters, beispielsweise Dibenzoylperoxyd, aber auch in Gegenwart von Licht durchgeführt werden (siehe z.B.: *J. Org. Chem.* **1949,** *14,* 433; *Bull. Chem. Soc. Jpn*. **1986,** *59,* 3702; *Monatshefte Chem* **1975**, *106*, 1415). Es können auch andere Bromierungsreagenzien eingesetzt werden; zum Beispiel sei N,N-Dibromdimethylhydantoin genannt. Üblicherweise wird die Umsetzung in einem geeigneten Lösemittel wie etwa Benzol oder einem Gemisch aus Benzol und Hexan bei erhöhter Temperatur durchgeführt (siehe z.B.: *J. Med. Chem.* **1977,** *20, 5; J. Am. Chem. Soc*. **1977**, *99,* 3432). Für den Bromierungsschritt können auch andere als die bisher genannten Lösemittel eingesetzt werden, beispielsweise Ameisensäuremethylester (z.B.: *Angew. Chem*. **1980,** *92,* 471).

Zur Bromwasserstoffabspaltung können verschiedene Reagenzien verwendet werden, bevorzugt werden Stickstoffbasen wie etwa Chinaldin oder Collidin, aber auch andere Reagenzien, wie Trimethylphosphit. Die Umsetzung wird in geeigneten Lösemitteln durchgeführt, beispielsweise in einem aromatischen Kohlenwasserstoff wie Xylol in einem Temperaturbereich zwischen 70°C und 145°C (siehe z.B.: *Helv. Chim. Acta* **1973,** *56,* 1708; *J. Org. Chem.* **1951,** *16*, 1126: *J. Org. Chem.* **1982,** *47,* 2536).

Die Umsetzung einer Verbindung der Formel **5** zu einer Verbindung der Formel **6** kann aber auch durch direkte Dehydrierung in einem Reaktionsschritt erfolgen. Als Dehydrierungsmittel können Chinone, beispielsweise 2-Methyl-1,4-naphtochinon (*Recl. Trav. Chim. Pays-Bas* **1940,** *59*, 454) oder 1,4-Benzochinon (*J. Am. Chem. Soc.* **1946,** *68,* 738), verwendet werden. Bevorzugt für die Umsetzung einer Verbindung der Formel **5** zu einer Verbindung der Formel **6** werden jedoch die 2stufigen Verfahren, bestehend aus einem Bromierungs- und einem anschließenden Dehydrobromierungsschritt.

Die Isomerisierung einer Verbindung der Formel **6** (**→7**) kann nach verschiedenen Methoden erfolgen, beispielsweise kann Salzsäure in einem Lösemittelgemisch bestehend aus Ethanol, Benzol und Wasser verwendet werden (*J. Org. Chem.* **1986,** *51*, 4047). Auch sind Ethanol und Methanol als alleinige Lösemittel für solche Dien-Isomerisierungen beschrieben, wobei ebenfalls Salzsäure verwendet wird (z.B.: *J. Am. Chem.Soc*. **1953,** *75,* 4404; *Tet. Lett*. **1967,** 3699). Wird nach einer der zuvor beschriebenen Methoden gearbeitet, werden Verbindungen der Formel 7 erhalten, in der R² Wasserstoff und R¹ Ethyl oder Methyl bedeutet, je nach verwendetem Alkohol. Auch die Verwendung von HCl-Gas in Lösemitteln wie Chloroform oder Essigsäure ist beschrieben (z.B.: *J. Org. Chem*. **1988,** *53,* 1563; *J. Chem. Soc*. **1962,** 2917). Die Isomerisierung läßt sich aber auch unter Verwendung anderer Säuren und/oder Lösemittel durchführen, so mit p-Toluolsulfonsäure in Benzol *(Chem. Pharm. Bull*. **1988,** *36,* 2724).

Die Isomerisierung des 5,7-Diens läßt sich auch mit Schwefelsäure in Lösemitteln wie Dioxan, primären Alkoholen oder deren Gemischen mit und ohne Zusatz von aromatischen Kohlenwasserstoffen wie etwa Toluol bei erhöhter Temperatur durchführen, der bevorzugte Temperaturbereich reicht hier von 70°C bis 120°C, wobei gegebenenfalls in einem Druckgefäß gearbeitet wird. Hierbei wird eine Verbindung der Formel **7** erhalten, in der R² Wasserstoff bedeutet und R¹ dem Kohlenwasserstoffteil eines eventuell verwendeten Alkohols entspricht, ohne Alkoholzusatz bleibt R¹ in der Regel unverändert. Weiterhin kann die gewünschte Isomerisierung auch in Schwefeldioxyd bei erhöhter Temperatur im Druckgefäß durchgeführt werden (*J. Chem. Soc*. **1954,** 814). Ebenfalls beschrieben ist die Verwendung von Übergangsmetallkatalysatoren wie etwa Rhodiumtrichlorid (*J. Chem. Soc. Perkin I*, **1977,** 359).

Die Alkylierung einer Verbindung der Formel **7** (**→8**) wird bevorzugt an solchen Derivaten durchgeführt, in welchen R¹ Methyl oder Ethyl und R² Wasserstoff oder eine Schutzgruppe wie Trialkylsilyl, Tetrahydropyranyl, Methoxymethyl oder etwa Methoxyethoxymethyl bedeuten. Gegebenenfalls wird die gewünschte Schutzgruppe vor der Alkylierung nach dem Fachmann an sich bekannten Methoden eingeführt. Alkylierungen steroidaler 20-Carbonsäureester sind vielfältig beschrieben. Überwiegend werden hier der Methyl- oder Ethylester eingesetzt. Neben der häufig beschriebenen Einführung einer 20-Methylgruppe sind auch eine Reihe Alkylierungen mit komplexen Bausteinen beschrieben (siehe beispielsweise *Bull. Soc. Chim. Belg.* **1986,** *95,* 289; *Tet. Lett*. **1987,** *28*, 1685; *J. Am. Chem. Soc* **1995,** *117*, 1849; *J. Chem. Soc. Chem. Comm*., **1975,** 968).

Als Alkylierungsreagenz kann hier das 5-Bromo-2-methyl-2-penten oder das 5-Iodo-2-methyl-2-penten (z.B.: *Synthesis* **1979,** 37) oder ein Sulfonsäureester, bevorzugt der Methansulfonsäure- bzw. der *p*-Toluolsulfonsäureester des entsprechenden Carbinols 4-Methyl-3-pentenol eingesetzt. Zur Deprotonierung einer Verbindung der Formel **7** können verschiede Basen eingesetzt werden. Als Beispiele seien Kalium- und Natriumhexamethyldisilazid (*Tet. Lett.* **1996,** *37,* 7473; *Chem. Comm.* **1997,** *8,* 765) und auch andere Stickstoffbasen, beispielsweise Lithiumdiisopropylamid (siehe z.B. *J. Chem. Soc. Perkin I*, **1978,** 1282; *Tet. Lett.* **1996,** *37,* 9361) genannt. Auch andere Lithiumdialkylamidbasen können verwendet werden. Bevorzugt wird jedoch Lithiumdiisopropylamid. Mit oder auch nach Zugabe des Alkylierungsmittels kann der Reaktion Hexamethylphosphon- bzw. Hexamethylphosphorsäuretriamid zugesetzt werden. Als Lösemittel werden aprotische Solventien, bevorzugt Ether wie etwa Diethylether oder auch Tetrahydrofuran oder deren Gemische mit Kohlenwasserstoffen, z. B. Hexan, verwendet. Bevorzugt wird hier jedoch Tetrahydrofuran mit oder ohne Zusatz von Hexan. Die Reaktion wird in einem Temperaturbereich von -78°C bis Raumtemperatur durchgeführt, bevorzugt jedoch im Temperaturbereich von -40°C bis 10°C.

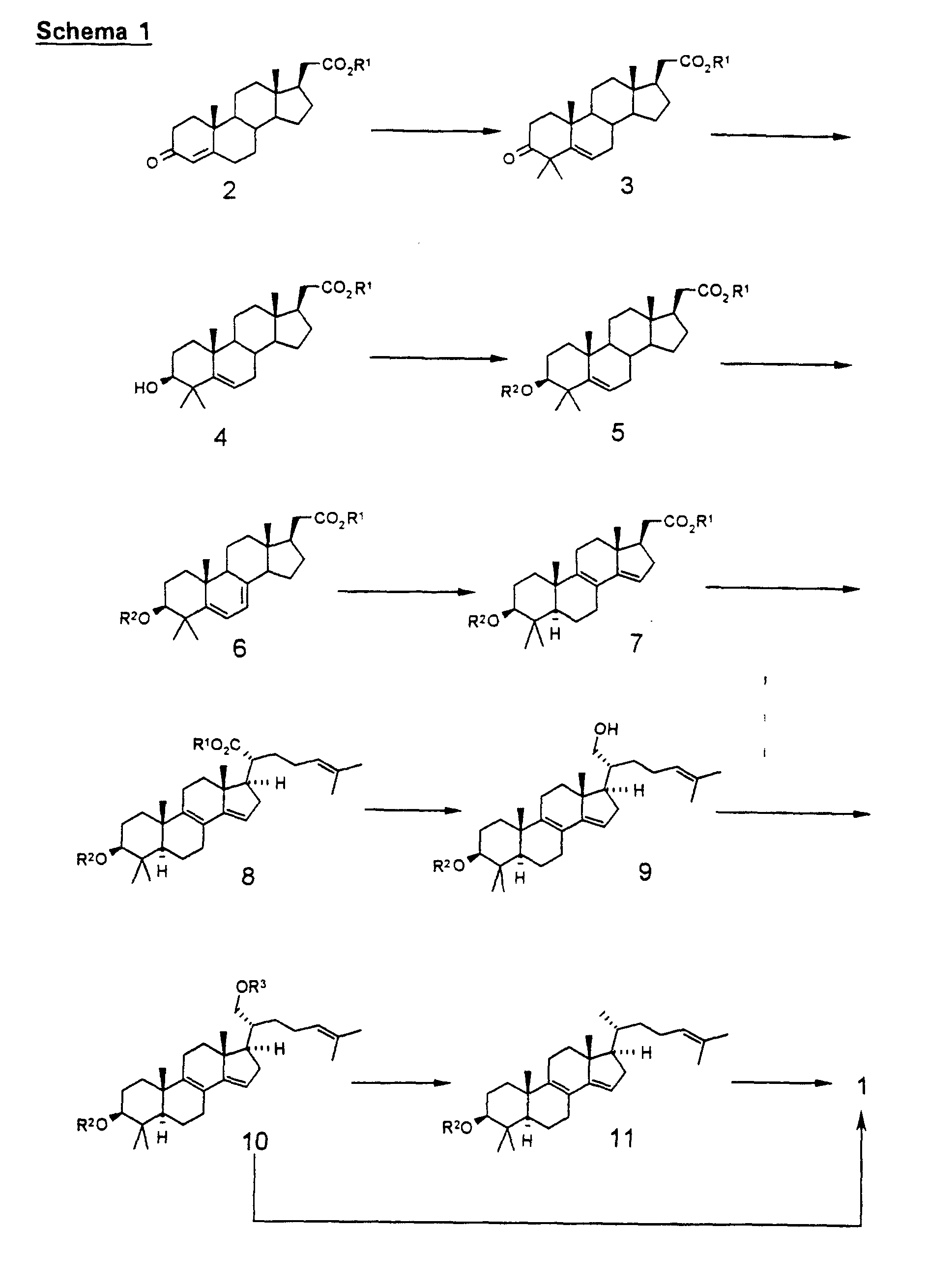

Zur Komplettierung der Synthese wird in einem Mehrstufenprozeß die Estergruppe einer Verbindung der Formel **8** zur Methylgruppe (Verbindungen der allgemeinen Formel **11**) reduziert. Die Reduktionssequenz besteht üblicherweise aus drei Schritten. Zunächst wird der Ester zu einem Alkohol der Formel **9** reduziert. Als Reduktionsmittel werden hier bevorzugt Lithiumaluminiumhydrid oder Diisobutylaluminiumhydrid in geeigneten aprotischen Lösemitteln wie beispielsweise Kohlenwasserstoffen, z.B. Toluol, oder Ethern, z.B. Tetrahydrofuran, oder deren Gemischen verwendet. Die Reaktionen werden in einem Temperaturbereich von -78°C bis 40°C durchgeführt, bevorzugt jedoch im Bereich von -40°C bis 25°C. Nach Überführung der Hydroxygruppe einer Verbindung der Formel **9** in eine Abgangsgruppe wird eine so erhaltene Verbindung der Formel **10** weiterreduziert. Die Wahl einer geeigneten Abgangspruppe für die Hydroxyfunktion einer Verbindung der Formel **10** hängt von der Natur des Substituenten R² ab. Bedeutet R² Wasserstoff, so muß ein Reagenz gewählt werden, welches eine Differenzierung zwischen der sekundären Hydroxylgruppe an C-3 und der primären Hydroxylgruppe an C-21 gewährleistet. Hierfür eignen sich insbesondere reaktive Sulfonsäurederivate sterisch anspruchsvoller Sulfonsäuren, beispielsweise die Anhydride oder Säurehalogenide der *p*-Toluolsulfonsäure oder der 2,4,6-Trimethylbenzolsulfonsäure, die zwischen primärer und sekundärer Hydroxylfunktion differenzieren. Handelt es sich bei R² um eine der angegebenen Schutzgruppen, können auch Derivate anderer Sulfonsäuren, etwa Methansulfonsäurechlorid, eingesetzt werden. Diese Veresterungen werden bevorzugt in Gegenwart einer Base wie Pyridin oder aliphatischen tertiären Aminen, beispielsweise Triethylamin, die als alleiniges Lösemittel verwendet werden können, durchgeführt. Die Reaktion kann aber auch unter Zusatz eines Lösemittels wie etwa Dichlormethan durchgeführt werden. Üblicherweise wird hier in einem Temperaturbereich von 0°C bis 70°C gearbeitet. Die Reduktion einer Verbindung der Formel **10** kann mit den gleichen Reagenzien und unter denselben Reaktionsbedingungen bewirkt werden wie zuvor für die Reduktion des Esters beschrieben. Als Reduktionsmittel sei hier zusätzlich Lithiumtriethylborhydrid genannt, welches sich insbesondere für die reduktive Entfernung von Sulfonsäureestern bewährt hat. Beispiele für solche mehrstufigen Überführungen eines Esters in eine Methylgruppe finden sich vielfältig in der Literatur, unter anderem in: *Tet. Lett*. **1987,** *28,* 1685; *J. Am. Chem. Soc*. **1995,** *117*, 1849 etc..

So wird für R² in der Bedeutung von Wasserstoff direkt FF-MAS (1) erhalten. Stellt R² jedoch eine Schutzgruppe dar (siehe oben), wird eine Verbindung der Formel **11** erhalten, aus welcher die Schutzgruppe nach dem Fachmann geläufigen Methoden abgespalten wird.

### Verfahrensvariante 2:

In dieser Verfahrensvariante (vgl. Schema 2) wird der Isomerisierungsschritt (5,7-Dien→8,14-Dien) an das Syntheseende verschoben . Die Sequenz Alkylierung und Reduktion der Estergruppe (**6→→15**) wird analog den in Verfahrensvariante 1 beschriebenen Methoden durchgeführt. Die Isomerisierung einer Verbindung der Formel **15** wird ebenfalls analog Verfahrensvariante 1 durchgeführt. Für R² in der Bedeutung von Wasserstoff wird direkt FF-MAS (1) erhalten. Stellt R² eine Schutzgruppe dar (siehe oben), so wird, sofern die betreffende Schutzgruppe unter den für die Isomerisierung verwendeten Reaktionsbedigungen erhalten bleibt, nicht direkt FF-MS (**1**) sondern eine Verbindung der Formel **11** erhalten, aus welcher die Schutzgruppe nach dem Fachmann geläufigen Methoden abgespalten wird.

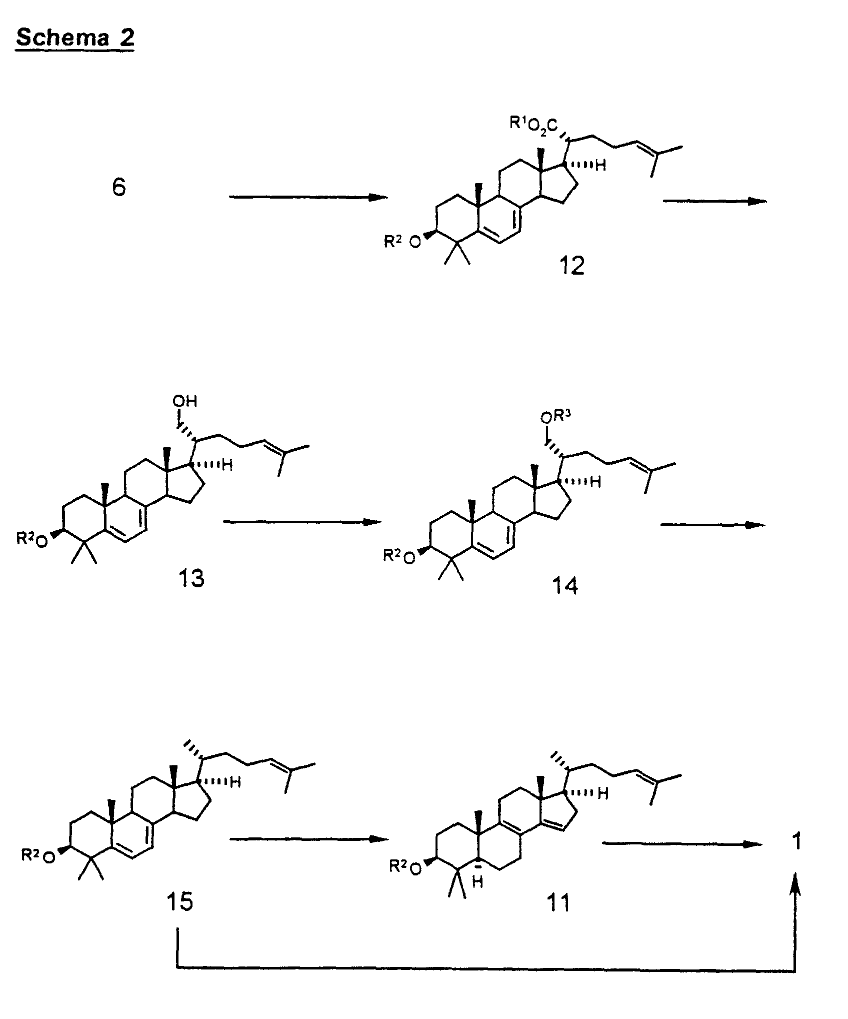

### Beispiele

### Beispiel 1 (Verfahrensvariante 2)

### a) 4,4-Dimethyl-3-oxopregn-5-en-21-säuremethylester:

Zu 186,6 g Kaliumtertiärbutylat in einem Liter tertiär-Butanol werden 143,2 g 3-Oxopregn-4-en-21-säuremethylester, gelöst in einem Liter Tetrahydrofuran, bei 45°C zugegeben. Nach 10 Minuten werden 183 ml Methyljodid zugetropft. Nach weiteren 50 Minuten wird auf 10 Liter Eiswasser gegossen, mit 4 normaler Salzsäure angesäuert und mit Ethylacetat extrahiert. Nach Waschen der organischen Phase mit Wasser, Natriumhydrogencarbonatlösung und gesättigter Kochsalzlösung wird über Natriumsulfat getrocket, filtriert, eingeengt und der Eindampfrückstand an Kieselgel mit einem Gemisch aus n-Hexan und Ethylacetat chromatographiert. Es werden 75,1 g 4,4-Dimethyl-3-oxopregn-5-en-21-säuremethylester erhalten.
¹H-NMR (CDCl₃): δ = 0,62 ppm (s, 3H, H-18); 0,87 (s, 3H, H-19); 1,22 (s, 6H, 4-CH₃); 2,14 (dd, J=10 und 15Hz, 1H, H-20); 2,39 (dd, J=5 und 15 Hz, 1H, H-20); 3,66 (s, 3H, CO₂-CH₃); 5,55 (m, 1H, H-6).

### b) 4,4-Dimethyl-3β-hydroxypregn-5-en-21-säuremethylester:

74,5 g der in Stufe a) beschriebenen Verbindung werden in einem Liter Dichlormethan vorgelegt und mit 7,57 g Natriumborhydrid versetzt. Nach dem Zutropfen von 0,1 Litern Methanol wird 4,5 Stunden gerührt. Das Reaktionsgemisch wird in einen Liter eiskalte 1 normale Salzsäure eingerührt und anschließend zwischen Ethylacetat und Wasser verteilt. Nach Phasentrennung, Waschen der organischen Phase mit gesättigter Kochsalzlösung, Trocknung über Natriumsulfat, Filtration und Einengen des Filtrats werden 75,2 g 4,4-Dimethyl-3β-hydroxypregn-5-en-21-säuremethylester erhalten, welcher ohne Reinigung weiterverwendet wird.
¹H-NMR (CDCl₃): δ = 0,60 ppm (s, 3H, H-18); 1,08 (s, 3H, 4-CH₃); 1,10 (s, 3H, 4-CH₃); 1,14 (s, 3H, H-19); 2,22 (dd, J=10 und 15 Hz, 1H, H-20); 2,38 (dd, J=5 und 15 Hz, 1H, H-20); 3,25 (m, 1H, H-3); 3,66 (s, 3H, CO₂-CH₃); 5,57 (m, 1H, H-6).

### c) 4,4-Dimethyl-3β-[[dimethyl(1,1-dimethylethyl)silyl]oxy]pregn-5-en-21-säuremethylester:

74,7 g der in Stufe b) beschriebenen Verbindung werden mit 40,8 g Imidazol und 60,3 g tertiär-Butyldimethylsilylchlorid in 1,25 Litern N,N-Dimethylformamid 20 Stunden bei 40°C gerührt. Anschließend wird auf 10 Liter 0,5 normale Salzsäure gegossen und abgenutscht. Der Filterkuchen wird mit 4 Litern 0,5 normaler Natronlauge ausgerührt. Nach erneutem Abnutschen und Trocknung des Filterkuchens werden 93,5 g 4,4-Dimethyl-3β-[[dimethyl(1,1-dimethylethyl)silyl]oxy]pregn-5-en-21-säuremethylester erhalten, welcher ohne Reinigung weiterverwendet wird.
¹H-NMR (CDCl₃): δ = 0,03 ppm (s, 3H, Si-CH₃); 0,04 ppm (s, 3H, Si-CH₃); 0,61 ppm (s, 3H, H-18); 0,90 ppm (s, 9H, Si-^{t}Bu); 1,04 (s, 3H); 1,07 (s, 3H); 1,09 (s, 3H); 2,13 (dd, J=10 und 15 Hz, 1H, H-20); 2,38 (dd, J=5 und 15 Hz, 1H, H-20); 3,21 (dd, J=5 und 12 Hz, 1H, H-3); 3,67 (s, 3H, CO₂-CH₃); 5,55 (m, 1H, H-6).

### d) 4,4-Dimethyl-3β-[[dimethyl(1,1-dimethylethyl)silyl]oxy]pregna-5,7-dien-21-säuremethylester:

93 g der in Stufe c) beschriebenen Verbindung werden mit 32,6 g 1,3-Dibrom-5,5-dimethylhydantoin in einem Gemisch aus je 0,75 Litern n-Hexan und Benzol 20 Minuten gekocht. Nach Abkühlung wird abgesaugt, das Filtrat eingeengt und der Eindampfrückstand mit 45 ml Trimethylphosphit in 0,9 Litern Xylol eine Stunde gekocht. Nach Einengen und Chromatographie an Kieselgel mit einem Gemisch aus n-Hexan und Ethylacetat werden 86,35 g 4,4-Dimethyl-3β-[[dimethyl(1,1-dimethylethyl)silyl]oxy]pregna-5,7-dien-21-säuremethylester erhalten.
¹H-NMR (CDCl₃): δ = 0,02 ppm (s, 3H, Si-CH₃); 0,04 ppm (s, 3H, Si-CH₃); 0,50 ppm (s, 3H, H-18); 0,89 ppm (s, 9H, Si-^{t}Bu); 0,95 (s, 3H, H-19); 1,08 (s, 3H, 4-CH₃); 1,12 (s, 3H, 4-CH₃); 2,13 (dd, J=10 und 15 Hz, 1H, H-20); 2,40 (dd, J=5 und 15 Hz, 1H, H-20); 3,33 (dd, J=5 und 12 Hz, 1H, H-3); 3,66 (s, 3H, CO₂-CH₃); 5,52 (m, 1H, H-7) 5,89; (d, J=6 Hz, 1H, H-6).

### e) 4,4-Dimethyl-3β-[[dimethyl(1,1-dimethylethyl)silyl]oxy]cholesta-5,7,24-trien-21-säuremethylester:

Zu einer Lösung von 0,268 mol Lithiumdiisopropylamid, hergestellt aus 168 ml einer 1,6 molaren Lösung von n-Butyllithium in Hexan und 45 ml Diisopropylamin in 90 ml Tetrahydrofuran werden bei -30°C 65 g der in Stufe d) beschriebenen Verbindnung, gelöst in 0,8 Litern Tetrahydrofuran, zugetropft. Nach einer Stunde werden bei dieser Temperatur 56 g 5-Iodo-2-methyl-2-penten in 75 ml Tetrahydrofuran zugesetzt. Anschließend wird auf 0°C erwärmt und 16 Stunden bei dieser Temperatur belassen. Nach Zugabe von 0,1 Litern 1 normaler Salzsäure wird mit 1,5 Litern eines Gemisches aus Hexan und Ethylacetat versetzt. Nach Phasentrennung wird die organische Phase mit Wasser und gesättigter Kochsalzlösung gewaschen, über Natriumsulfat getrocknet und eingeengt. Nach Grobfiltration über Kieselgel mit Hexan und Dichlormethan als Eluenten wird aus einem Gemisch von Ethanol und tertiär-Butylmethylether kristallisiert. Es werden 41 g 4,4-Dimethyl-3β-[[dimethyl(1,1-dimethylethyl)silyl]oxy]cholesta-5,7,24-trien-21-säuremethylester erhalten.
¹H-NMR (CDCl₃): δ = 0,04 ppm (s, 3H, Si-CH₃); 0,07 ppm (s, 3H, Si-CH₃); 0,61 ppm (s, 3H, H-18); 0,91 ppm (s, 9H, Si-^{t}Bu); 0,97 (s, 3H, H-19); 1,10 (s, 3H, 4-CH₃); 1,13 (s, 3H, 4-CH₃); 1,58 und 1,70 (je 1s br., jeweils 3H, H-26 und H-27); 2,28 (m, 1H, H-20); 3,36 (dd, J=5 und 12 Hz, 1H, H-3); 3,68 (s, 3H, CO₂-CH₃); 5,08 (m, 1H, H-24); 5,53 (m, 1H, H-7); 5,90 (d, J=6 Hz, 1H, H-6).

### f) 4,4-Dimethyl-3β-[[dimethyl(1,1-dimethylethyl)silyl]oxy]cholesta-5,7,24-trien-21- 1-ol:

Zu 5,31 g Lithiumaluminiumhydrid, suspendiert in 0,25 Litern Tetrahydrofuran werden bei 0°C 40 g der in Stufe e) beschriebenen Verbindung in 0,25 Litern Tetrahydrofuran getropft.
Nach 3 Stunden Rühren bei Raumtemperatur wird unter Eiskühlung mit 20 ml gesättigter Ammoniumchloridlösung versetzt. Nach 10 Minuten wird mit Natriumsulfat versetzt und nach weiteren 5 Minuten abgesaugt. Nach Einengen des Filtrats werden 37,57 g 4,4-Dimethyl-3β-[[dimethyl(1,1-dimethylethyl)silyl]oxy]cholesta-5,7,24-trien-21-ol erhalten.
¹H-NMR (CDCl₃): δ = 0,04 ppm (s, 3H, Si-CH₃); 0,06 ppm (s, 3H, Si-CH₃); 0,61 ppm (s, 3H, H-18); 0,90 ppm (s, 9H, Si-^{t}Bu); 0,98 (s, 3H, H-19); 1,10 (s, 3H, 4-CH₃); 1,13 (s, 3H, 4-CH₃); 1,62 und 1,70 (je 1s br., jeweils 3H, H-26 und H-27); 3,36 (dd, J=5 und 12 Hz, 1H, H-3); 3,73 (m, 2H, H-21); 5,12 (m, 1H, H-24); 5,55 (m, 1H, H-7); 5,91 (d, J=6 Hz, 1H, H-6).

### g) 4,4-Dimethyl-3β-[[dimethyl(1,1-dimethylethyl)silyl]oxy]cholesta-5,7,24-trien-21-ol-methansulfonat:

Bei 0°C werden zu einer Lösung von 36,5 g der in Stufe f) beschriebenen Verbindung in einem Gemisch aus 155 ml Dichlormethan und 30 ml Triethylamin 7,9 ml Methansulfonsäurechlorid zugetropft. Nach 3 Stunden bei Raumtemperatur wird zwischen Wasser und Dichlormethan verteilt. Nach Waschen der organischen Phase mit Natriumhydrogencarbonatlösung, gesättigter Kochsalzlösung, Trockung über Natriumsulfat, Filtration und Einengen des Filtrates werden 47,4 g rohes 4,4-Dimethyl-3β-[[dimethyl(1,1-dimethylethyl)silyl]oxy]cholesta-5,7,24-trien-21-olmethansulfonat erhalten, welches ohne Reinigung weiterverwendet wird.
¹H-NMR (CDCl₃): δ = 0,04 ppm (s, 3H, Si-CH₃); 0,06 ppm (s, 3H, Si-CH₃); 0,62 ppm (s, 3H, H-18); 0,89 ppm (s, 9H, Si-^{t}Bu); 0,97 (s, 3H, H-19); 1,09 (s, 3H, 4-CH₃); 1,12 (s, 3H, 4-CH₃); 1,61 und 1,70 (je 1s br., jeweils 3H, H-26 und H-27); 3,02 (s, 3H, OSO₂-CH₃); 3,36 (dd, J = 5 und 12 Hz, 1H, H-3); 4,24 (dd, J = 5 und 10 Hz, 1H, H-21); 4,39 (dd, J = 3 und 10 Hz, 1H, H-21); 5,09 (m, 1H, H-24); 5,55 (m, 1H, H-7); 5,91 (d, J=6 Hz, 1H, H-6).

### h) 4,4-Dimethyl-3β-[[dimethyl(1,1-dimethylethyl)silyl]oxy]cholesta-5,7,24-trien:

47,4 g des Rohprodukts aus Stufe g) werden nach der in Stufe f) beschriebenen Methode umgesetzt. Nach Chromatographie des Rohprodukts an Kieselgel mit n-Hexan als Eluent werden 31 g 4,4-Dimethyl-3β-[[dimethyl(1,1-dimethylethyl)-silyl]oxy]cholesta-5,7,24-trien erhalten.
¹H-NMR (CDCl₃): δ = 0,04 ppm (s, 3H, Si-CH₃); 0,06 ppm (s, 3H, Si-CH₃); 0,60 ppm (s, 3H, H-18); 0,90 ppm (s, 9H, Si-^{t}Bu); 0,98 (d, J-7 Hz, 3H, H-21); 0,99 (s, 3H, H-19); 1,10 (s, 3H, 4-CH₃); 1,13 (s, 3H, 4-CH₃); 1,60 und 1,70 (je 1s br., jeweils 3H, H-26 und H-27); 3,35 (dd, J=5 und 12 Hz, 1H, H-3); 5,10 (m, 1H, H-24); 5,53 (m, 1H, H-7); 5,90 (d, J=6 Hz, 1H, H-6).

### i) 4,4-Dimethyl-5α-cholesta-8,14,24-trien-3β-ol:

22 g der in Stufe h) beschriebenen Verbindung werden in 2,2 Litern 1,4-Dioxan mit 110 ml 6 normaler Schwefelsäure 170 Stunden gekocht. Nach weitgehendem Abziehen des Lösemittels wird der Eindampfrückstand zwischen Natriumhydrogencarbonatlösung und Ethylacetat verteilt. Nach Waschen der organischen Phase mit Natriumhydrogencarbonatlösung und gesättigter Kochsalzlösung, Trocknung über Natriumsulfat, Filtration und Einengen wird der Eindampfrückstand an Kieselgel mit einem Gemisch aus Hexan und Ethylacetat chromatographiert. Nach Einengen des Eluats und Kristallisation des Eindampfrückstandes aus Ethanol werden 4,3 g 4,4-Dimethyl-5α-cholesta-8,14,24-trien-3β-ol in 90 %iger Reinheit erhalten. Rechromatographie der Mutterlaugen, Einengen des Eluats und Kristallisation des Eindampfrückstandes aus einem Methanol-Wasser Gemisch ergeben weitere 3,8 g 4,4-Dimethyl-5α-cholesta-8,14,24-trien-3β-ol.
Insgesamt werden so 8,1 g (20 mmol) 4,4-Dimethyl-5α-cholesta-8,14,24-trien-3β-ol erhalten.

Die NMR-Daten stimmen mit denen der Literatur überein *(J. Am. Chem. Soc. 111,* **1989,** 278).

### Beispiel 2 (Verfahrensvariante 1)

### a) 4,4-Dimethyl-3β-hydroxypregna-8,14-dien-21-säuremethylester

300 g der in Beispiel 1, Stufe d) beschriebenen Verbindung werden in einem Gemisch aus 2,7 Litern Methanol und 0,4 Liter konzentrierter Salzsäure 20 Stunden gekocht. Nach Kühlung im Eisbad wird vom Kristallisat abgesaugt, das Filtrat zwischen Dichlormethan und Wasser verteilt und die organische Phase mit Wasser neutral gewaschen. Nach Waschen mit gesättigter Kochsalzlösung, Trocknung über Natriumsulfat und Filtration wird eingeengt und über eine Filtersäule von Silanolen abgetrennt. Das Kristallisat wird mit Wasser gewaschen und getrocknet. Es werden 125 g Kristallisat und 96 g Mutterlauge erhalten. Das Kristallisat wird erneut in einem Gemisch aus 1,2 Liter Methanol und 0,2 Liter konzentrierter Salzsäure 24 Stunden gekocht. Nach Filtration des erkalteten Reaktionsgemisches wird filtriert, die Mutterlauge gewaschen, getrocknet und eingeengt. Es werden 77 g Kristallisat und 46 g Mutterlauge erhalten. Chromatographie der so erhaltenen Mutterlaugen und des zweiten Kristallisates an Kieselgel mit einem Gemisch aus n-Hexan und Ethylacetat ergeben 92 g 4,4-Dimethyl-3β-hydroxypregna-8,14-dien-21-säuremethylester.

### b) 4,4-Dimethyl-3β-[[dimethyl(1,1-dimethylethyl)silyl]oxy]pregna-8,14-dien-21-säuremethylester

92 g der in Stufe a) beschriebenen Verbindung werden mit 0,75 Litern N,N-Dimethylformamid, 51 g tertiär-Butyldimethylsilylchlorid und 27,8 g Imidazol 18 Stunden bei 70°C gerührt. Nach Abkühlung wird auf 10 Liter einer eiskalten 0,5 molaren wässrigen Salzsäure gegossen und filtriert. Der Filterkuchen wird in Ethylacetat aufgenommen, mit 1 normaler Natronlauge neutral gewaschen, über Natriumsulfat getrocknet, filtriert und eingeengt. Es werden 124,8 g 4,4-Dimethyl-3β-[[dimethyl(1,1-dimethylethyl)silyl]oxy]pregna-8,14-dien-21-säuremethylester erhalten, welcher ohne Reinigung weiterverwendet wird.

### c) 4,4-Dimethyl-3β-[[dimethyl(1,1-dimethylethyl)silyl]oxy]-5β-cholesta-8,14,24-trien-21-säuremethylester

Zu einer Lösung von 1,04 mol Lithiumdiisopropylamid, hergestellt aus 652 ml einer 1,6 molaren Lösung von n-Butyllithium in Hexan und 174 ml Diisopropylamin in 320 ml Tetrahydrofuran werden bei -20°C 123,5 g der in Stufe b) beschriebenen Verbindung, gelöst in 2,0 Litern Tetrahydrofuran, zugetropft. Nach 40 Minuten Rühren bei 0°C wird auf -10°C gekühlt, und es werden 270 g 5-Iodo-2-methyl-2-penten zugetropft. Nach 3stündigem Rühren bei 0°C wird der Ansatz zwischen Ethylacetat und gesättigter Ammoniumchloridlösung verteilt. Nach Waschen der organischen Phase mit Wasser und gesättigter Kochsalzlösung, Trockung über Natriumsulfat und Filtration wird eingeengt und grob über Kieselgel mit einem Gemisch aus n-Hexan und Ethylacetat filtriert. Es werden 113 g (0,2 mol) 4,4-Dimethyl-3β-[[dimethyl(1,1-dimethylethyl)silyl]oxy]-5α-cholesta-8,14,24-trien-21-säuremethylester erhalten, welcher ohne Reinigung weiterverwendet wird.

### d) 4,4-Dimethyl-3β-[[dimethyl(1,1-dimethylethyl)silyl]oxy]-5α-cholesta-8,14,24-trien-21-ol

Zu 15,04 g Lithiumaluminiumhydrid, suspendiert in 0,7 Liter Tetrahydrofuran werden bei 0°C 112,5 g der in Stufe c) beschriebenen Verbindung, gelöst in 0,7 Liter Tetrahydrofuran, getropft. Nach 3 Stunden Rühren bei Raumtemperatur wird unter Eiskühlung mit 60 ml gesättigter Ammoniumchloridlösung versetzt. Nach 20 Minuten Rühren wird mit Natriumsulfat versetzt und nach weiteren 10 Minuten abgesaugt. Der Eindampfrückstand wird über eine kurze Säule mit Dichlormethan als Lösemittel filtriert. Nach Einengen des Eluats werden 103,2 g 4,4-Dimethyl-3β-[[dimethyl(1,1-dimethylethyl)silyl]oxy]-5α-cholesta-8,14,24-trien-21-ol erhalten, welches ohne weitere Reinigung weiterverwendet wird.

### e) 4,4-Dimethyl-3β-[[dimethyl(1,1-dimethylethyl)silyl]oxy]-5α-cholesta-8,14,24-trien-21-ol-methansulfonat

Bei 0°C werden zu einer Lösung von 102,3 g der in Stufe d) beschriebenen Verbindung in einem Gemisch aus 440 ml Dichlormethan und 84 ml Triethylamin 21,8 ml Methansulfonsäurechlorid getropft. Nach 3 Stunden bei Raumtemperatur wird zwischen Wasser und Dichlormethan verteilt. Nach Waschen der organischen Phase mit Natriumhydrogencarbonatlösung, gesättigter Kochsalzlösung, Trocknung über Natriumsulfat, Filtration und Einengen wird an Kieselgel mit einem Gemisch aus Hexan und Ethylacetat chromatographiert. Es werden 78,2 g 4,4-Dimethyl-3β-[[dimethyl(1,1-dimethylethyl)silyl]oxy]-5α-cholesta-8,14,24-trien-21-ol-methansulfonat erhalten.

### f) 4,4-Dimethyl-3β-[[dimethyl(1,1-dimethylethyl)silyl]oxy]-5α-cholesta-8,14,24-trien

77,2 g der in Stufe e) beschriebenen Verbindung werden nach der in Stufe d) beschriebenen Methode umgesetzt. Nach Filtration des Rohproduktes über Kieselgel mit einem Gemisch aus n-Hexan und Ethylacetat werden 63 g 4,4-Dimethyl-3β-[[dimethyl(1,1-dimethylethyl)silyl]oxy]-5α-cholesta-8,14,24-trien erhalten.

### g) 4,4-Dimethyl-5α-cholesta-8,14,24-trien-3β-ol

2 g der in Stufe f) beschriebenen Verbindung werden in einem Gemisch aus 5 ml 6 normaler Salzsäure, 10 ml Ethanol und 30 ml Tetrahydrofuran 24 Stunden bei Raumtemperatur gerührt. Anschließend wird zwischen Ethylacetat und Wasser verteilt. Nach Waschen der organischen Phase mit 1 normaler Natronlauge, Wasser und gesättigter Kochsalzlösung, Trocknung über Natriumsulfat und Filtration wird der Eindampfrückstand an Kieselgel mit einem Gemisch aus n-Hexan und Ethylacetat chromatographiert. Es werden 1,45 g 4,4-Dimethyl-5α-cholesta-8,14,24-trien-3β-ol erhalten.

Die NMR-Daten stimmen mit denen der Literatur überein (*J. Am. Chem. Soc. 111,* **1989,** 278).

## Patentansprüche

1. Verfahren zur Herstellung von 4,4-Dimethyl-5α-cholesta-8,14,24-trien-3β-ol der Formel 1 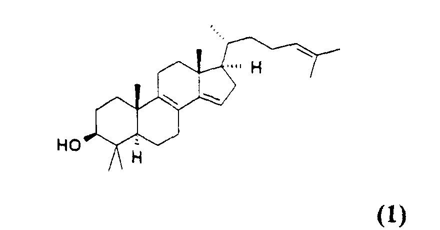 aus 3-Oxopregn-4-en-21-säureesterderivate der allgemeinen Formel 2 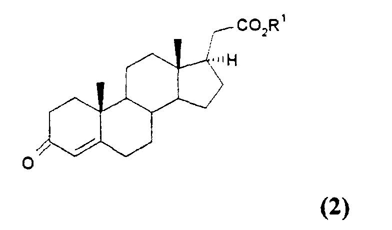 worin
R¹ Wasserstoff, verzweigtes oder unverzweigtes C₁-C₆ Alkyl, Phenyl, Benzyl, ortho-, meta- oder para-Methylphenyl bedeuten,
a) durch Umsetzung in Gegenwart von Basen und einem Methylierungsmittel zu Verbindungen der allgemeinen Formel **3** 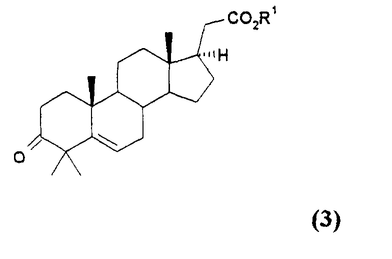 worin
R¹ die o.g. Bedeutung besitzt,
b) durch Reduktion zu Verbindungen der allgemeinen Formel 4 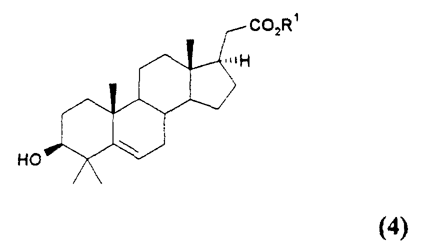 worin
R¹ die o.g. Bedeutung besitzt,
c) durch Überführung in eine 3-geschützte Hydroxygruppe der Verbindungen der allgemeinen Formel 5 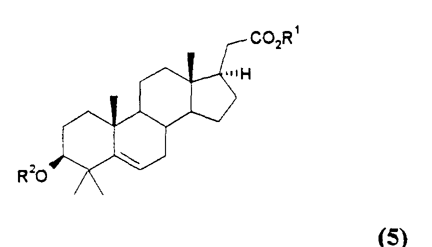 worin
R¹ die o.g. Bedeutung besitzt und
R² Ester aliphatischer und aromatischer Carbonsäuren, Acetalschutzgruppen, Etherschutzgruppe oder Silylether bedeuten,
d) durch Dehydrierung zu Verbindungen der allgemeinen Formel 6 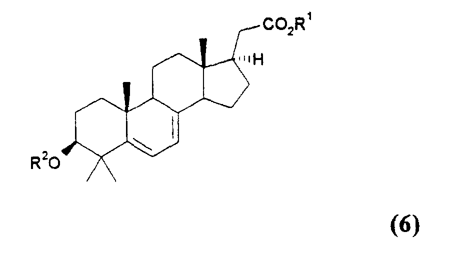 worin
R¹ und R² die o.g. Bedeutungen besitzen,
e) durch Isomerisierung zu Verbindungen der allgemeinen Formel 7 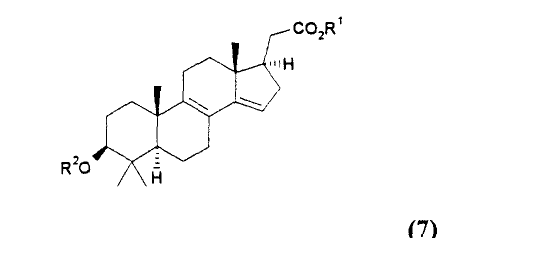 worin
R¹ und R² die o.g. Bedeutungen besitzen worin R² auch Wasserstoff sein kann
(e') gegebenfalls wird die gewünschte Schutzgruppe vor dem Alkylierungschritt (f) eingeführt
f) durch Alkylierung zu Verbindungen der allgemeinen Formel 8 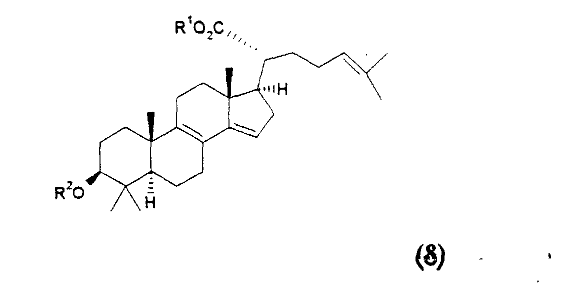 worin
R¹ und R² die o.g. Bedeutungen besitzen worin R² auch Wasserstoff sein kann,
g) durch Reduktion des Esters zu einer Hydroxymethylgruppe der Verbindungen der allgemeinen Formel 9 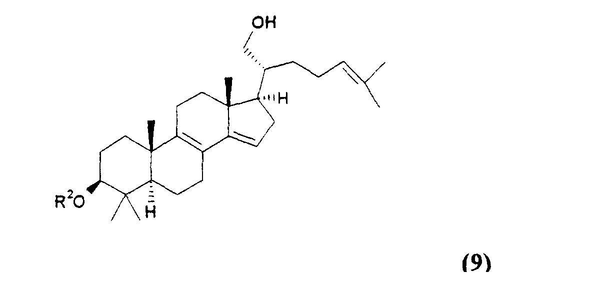 worin
R² die o.g. Bedeutung besitzt, oder Wasserstoff ist.
h) durch Überführung der primären Hydroxylgruppe in eine Abgangsgruppe in Verbindungen der allgemeinen Formel 10 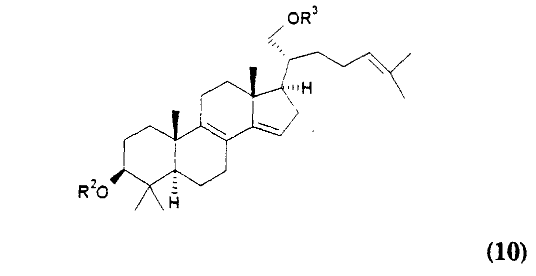 worin
R² die o.g. Bedeutung besitzt oder Wasserstoff ist und
R³ für den Rest SO₂R^{4,} steht, wobei R⁴ verzweigtes oder unverzweigtes C₁-C₆ Alkyl, Phenyl, Benzyl, ortho-, meta- oder para-Methylphenyl oder 2,4,6-Trimethylphenyl bedeutet,
und
i) durch reduktive Entfernung der -OR³ - Gruppierung in Verbindungen der allgemeinen Formel 11 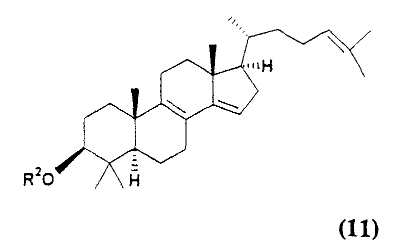 worin
R² die o.g. Bedeutung besitzt oder Wasserstoff ist und Abspaltung der Schutzgruppe, wenn R² eine Schutzgruppe darstellt.

2. Verfahren zur Herstellung von 4,4-Dimethyl-5α-cholesta-8,14,24-trien-3β-ol der Formel **1** 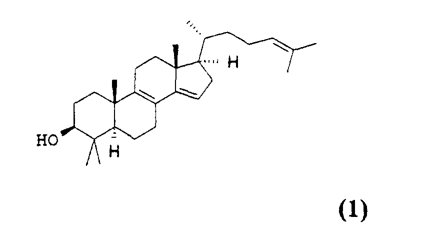 aus Verbindungen der allgemeinen Formel 6 nach Anspruch 1 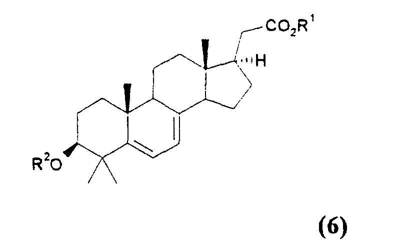 worin
R¹ und R² die in Schritt (c) von Anspruch 1 angegebenen Bedeutungen besitzen,
j) durch Alkylierung zu den Verbindungen der allgemeinen Formel 12 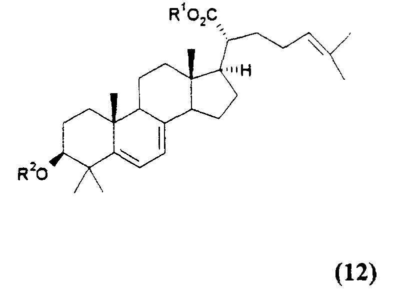 worin
R¹ und R² die o.g. Bedeutungen besitzen,
k) Reduktion der Estergruppe zur Hydroxymethylverbindung der allgemeinen Formel 13 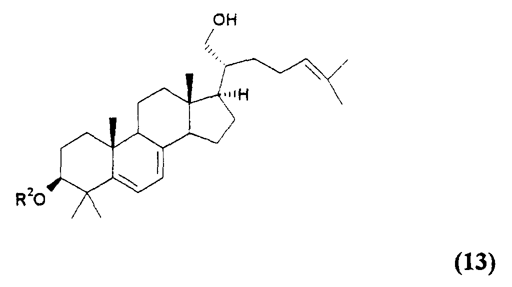 worin
R² die o.g. Bedeutung besitzt,
l) durch Überführung der primären Hydroxylgruppe in eine Abgangsgruppe in Verbindungen der allgemeinen Formel 14 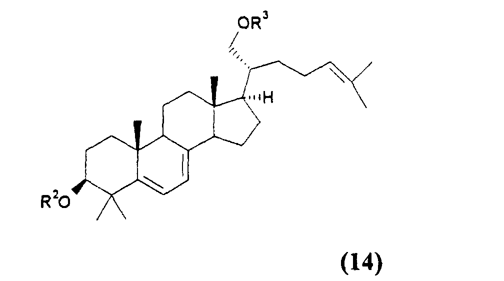 worin
R² die o.g. Bedeutungen besitzt, und R³ die in Anspruch I genannten Bedeutungen besitzt
m) durch reduktive Entfernung der -OR³- Gruppierung in Verbindungen der allgemeinen Formel 15 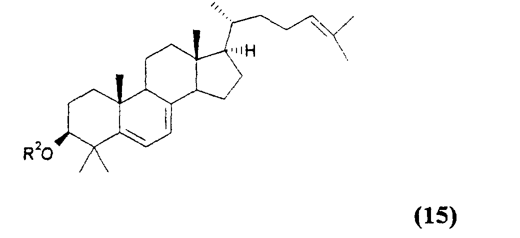 worin
R² die o.g. Bedeutung besitzt,
n) durch Isomerisierung in Verbindungen der allgemeinen Formel 11 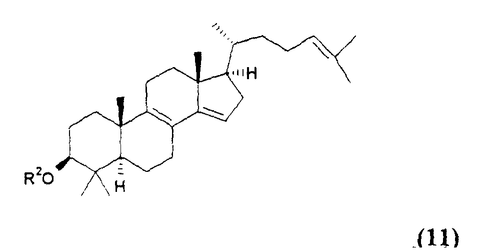 worin
R² die o.g. Bedeutung besitzt oder Wasserstoff ist, und Spaltung der Schutzgruppe, wenn R² eine Schutzgruppe darstellt.

3. Verbindungen der allgemeinen Formel **3** 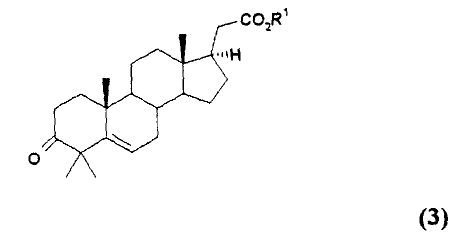 worin
R¹ Wasserstoff, verzweigtes oder unverzweigtes C₁-C₆ Alkyl, Phenyl, Benzyl, ortho-, meta- oder para-Methylphenyl bedeuten.

4. Verbindungen der allgemeinen Formel 4 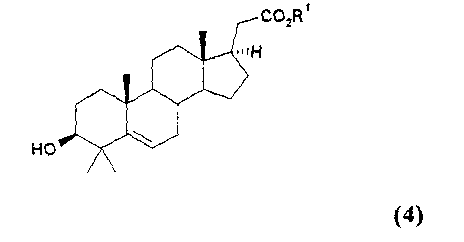 worin
R¹ Wasserstoff, verzweigtes oder unverzweigtes C₁-C₆ Alkyl, Phenyl, Benzyl, ortho-, meta- oder para-Methylphenyl bedeuten.

5. Verbindungen der allgemeinen Formel 5 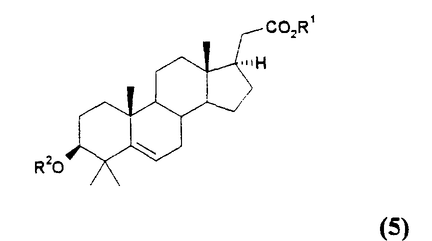 worin
R¹ verzweigtes oder unverzweigtes C₁-C₆ Alkyl, Phenyl, Benzyl, ortho-, meta- oder para-Methylphenyl und
R² Wasserstoff, Ester aliphatischer und aromatischer Carbonsäuren, oder Silylether bedeuten.

6. Verbindungen der allgemeinen Formel 6 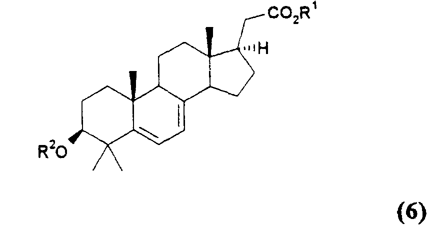 worin
R¹ verzweigtes oder unverzweigtes C₁-C₆ Alkyl, Phenyl, Benzyl, ortho-, meta- oder para-Methylphenyl und
R² Wasserstoff, Ester aliphatischer und aromatischer Carbonsäuren, oder Silylether bedeuten.

7. Verbindungen der allgemeinen Formel 7 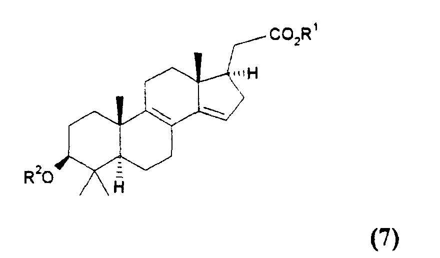 worin
R¹ Wasserstoff, verzweigtes oder unverzweigtes C₁-C₆ Alkyl, Phenyl, Benzyl, ortho-, meta- oder para-Methylphenyl und
R² Wasserstoff, Ester aliphatischer und aromatischer Carbonsäuren, oder Silylether bedeuten.

8. Verbindungen der allgemeinen Formel 8 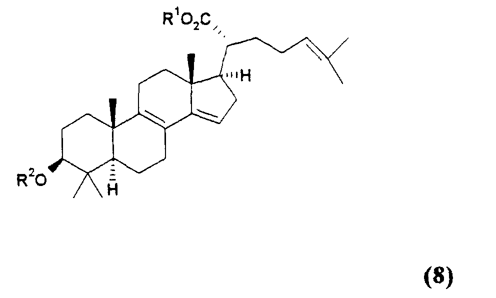 worin
R¹ Wasserstoff, verzweigtes oder unverzweigtes C₁-C₆ Alkyl, Phenyl, Benzyl, ortho-, meta- oder para-Methylphenyl und
R² Wasserstoff, Ester aliphatischer und aromatischer Carbonsäuren, Acetalschutzgruppen oder Silylether bedeuten.

9. Verbindungen der allgemeinen Formel 9 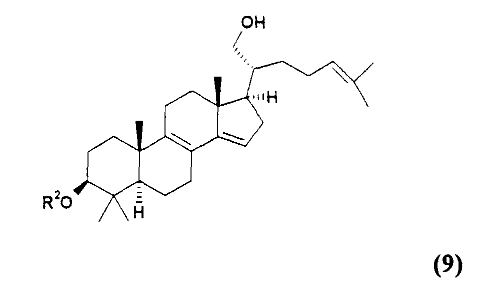 worin
R² Wasserstoff, Ester aliphatischer und aromatischer Carbonsäuren, Acetalschutzgruppen oder Silylether bedeuten.

10. Verbindungen der allgemeinen Formel 10 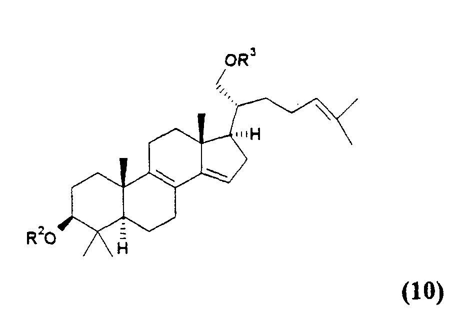 worin
R² Wasserstoff, Ester aliphatischer und aromatischer Carbonsäuren, Acetalschutzgruppen oder Silylether und
R³ SO₂R⁴, wobei R⁴ verzweigtes oder unverzweigtes C₁-C₆ Alkyl, Phenyl, Benzyl, ortho-, meta- oder para-Methylphenyl oder 2,4,6-Trimethylphenyl bedeuten.

11. Verbindungen der allgemeinen Formel 12 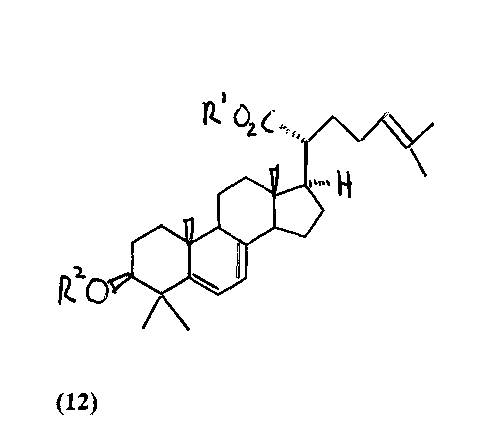 worin
R¹ verzweigtes oder unverzweigtes C₁-C₆ Alkyl, Phenyl, Benzyl; ortho- meta- oder para-Methylphenyl und
R² Wasserstoff, Ester aliphatischer und aromatischer Carbonsäuren, Acetalschutzgruppen oder Silylether bedeuten.

12. Hydroxymethylverbindung der allgemeinen Formel 13 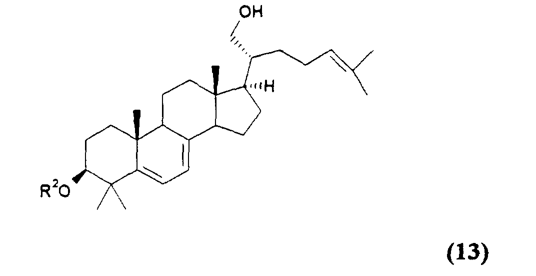 worin
R² Ester aliphatischer und aromatischer Carbonsäuren, Acetalschutzgruppen oder Silylether bedeuten.

13. Verbindungen der allgemeinen Formel 14 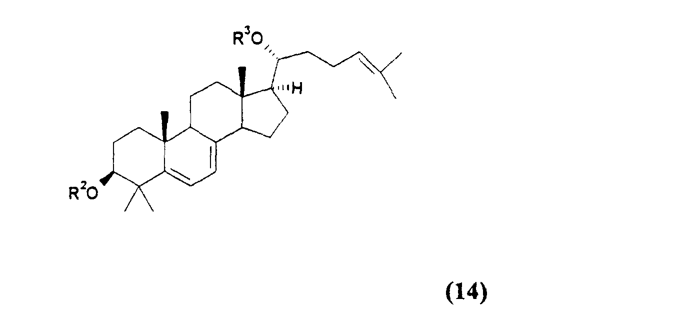 worin
R² Ester aliphatischer und aromatischer Carbonsäuren, Acetalschutzgruppen oder Silylether,
R³ SO₂R⁴, wobei R⁴ verzweigtes oder unverzweigtes C ₁-C₆ Alkyl, Phenyl, Benzyl, ortho-, meta- oder para-Methylphenyl oder 2,4, 6-Trimethylphenyl bedeuten.

14. Verbindungen der allgemeinen Formel 15 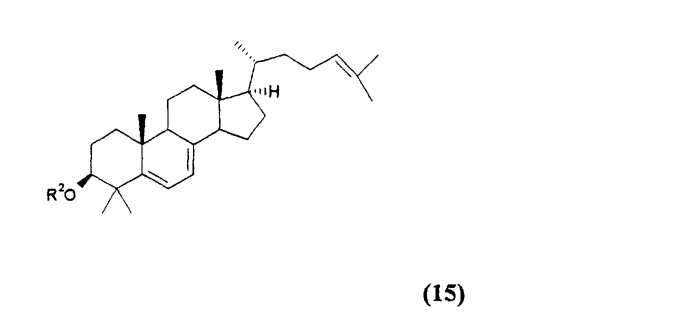 worin
R² Ester aliphatischer und aromatischer Carbonsäuren, Acetalschutzgruppen oder Silylether bedeuten.

## Claims

1. Process for the preparation of 4,4-dimethyl-5α-cholesta-8,14,24-trien-3β-ol of the formula I 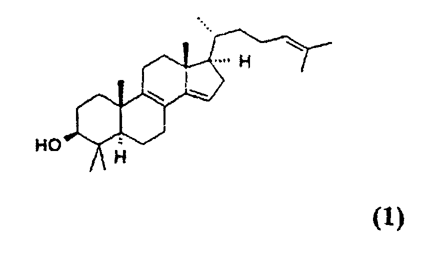 from 3-oxopregn-4-en-21-oic acid ester derivatives of the general formula 2 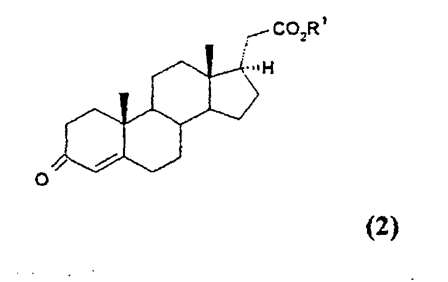 in which
R¹ is hydrogen, branched or unbranched C₁-C₆ alkyl, phenyl, benzyl, ortho-, meta- or para-methylphenyl,
a) by reaction in the presence of bases and a methylating agent to give compounds of the general formula 3 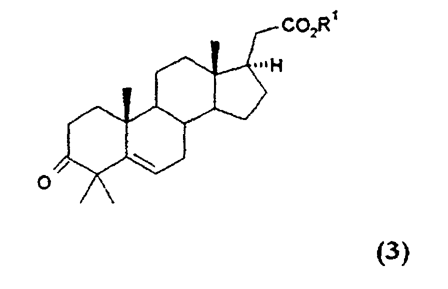 in which
R¹ has the abovementioned meaning,
b) by reduction to give compounds of the general formula 4 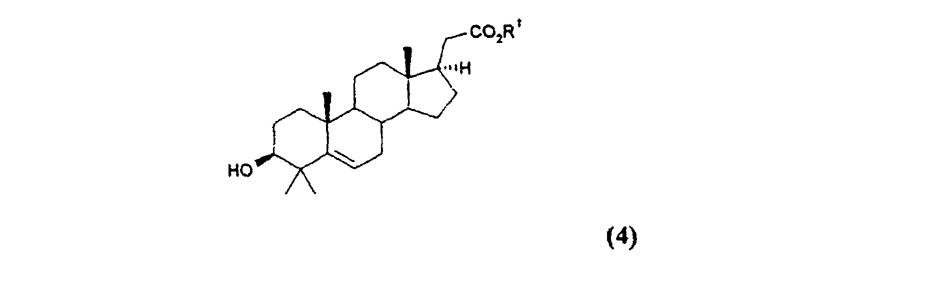 in which
R¹ has the abovementioned meaning,
c) by conversion to a 3-protected hydroxy group of the compounds of the general formula 5 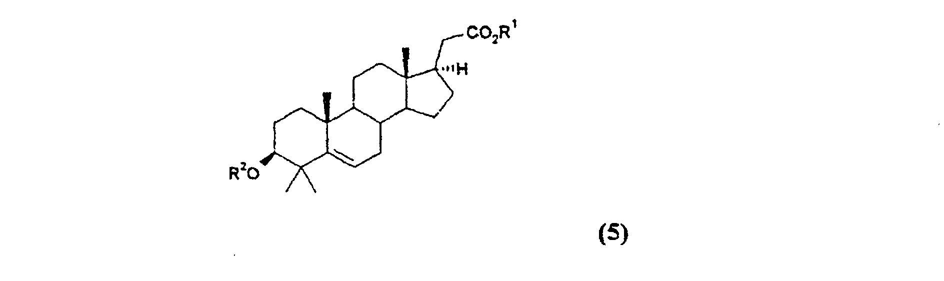 in which
R¹ has the abovementioned meaning and
R² is esters of aliphatic and aromatic carboxylic acids, acetal protective groups, ether protective groups or silyl ethers,
d) by dehydrogenation to give compounds of the general formula 6 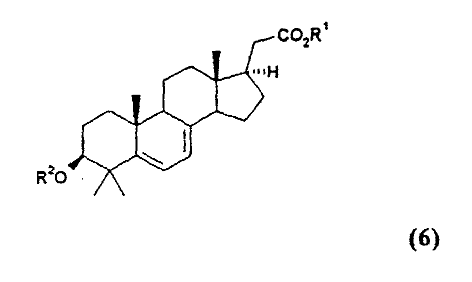 in which
R¹ and R² have the abovementioned meanings,
e) by isomerization to give compounds of the general formula 7 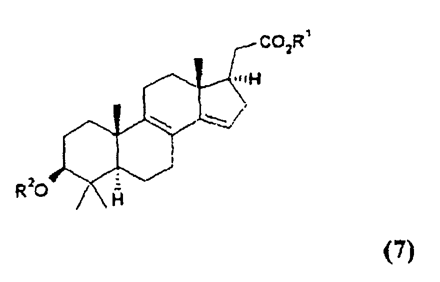 in which
R¹ and R² have the abovementioned meanings in which R² can also be hydrogen,
e') optionally, the desired protective group is introduced before the alkylation step (d)
f) by alkylation to give compounds of the general formula 8 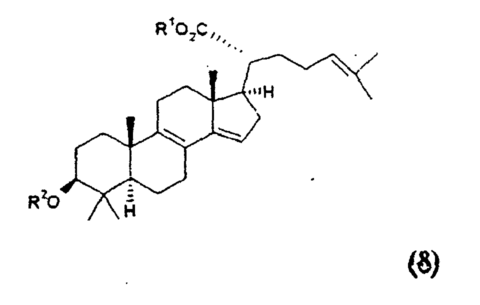 in which
R¹ and R² have the abovementioned meanings in which R² can also be hydrogen,
g) by reduction of the ester to a hydroxymethyl group of the compounds of the general formula 9 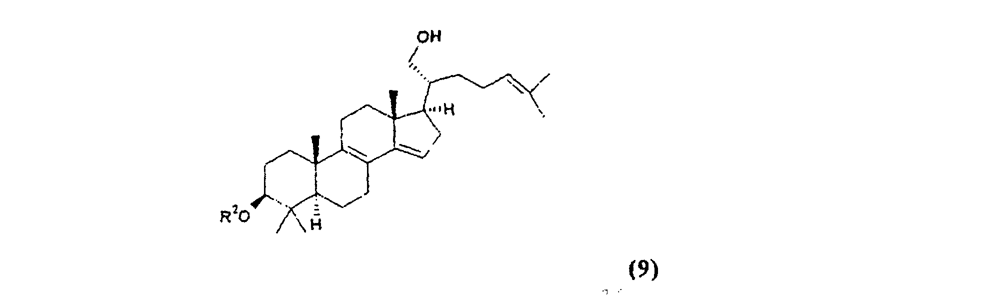 in which
R² has the abovementioned meaning or is hydrogen,
h) by conversion of the primary hydroxyl group to a leaving group in compounds of the general formula 10 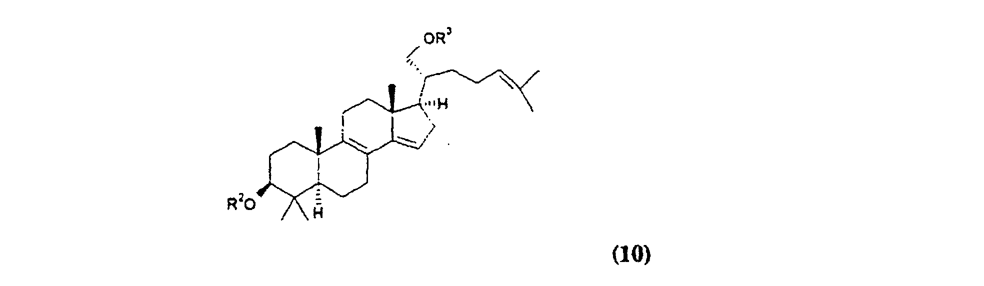 in which
R² has the abovementioned meaning or is hydrogen and
R³ is the radical SO₂R⁴, where R⁴ is branched or unbranched C₁-C₆ alkyl, phenyl, benzyl, ortho-, meta- or para-methylphenyl or 2,4,6-trimethylphenyl,
and
i) by reductive removal of the -OR³ group in compounds of the general formula 11 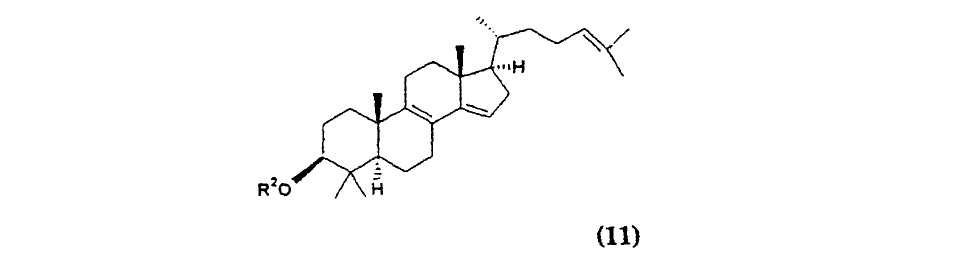 in which
R² has the abovementioned meaning and removal of the protective group if R² is a protective group.

2. Process for the preparation of 4,4-dimethyl-5α-cholesta-8,14,24-trien-3β-ol of the formula I 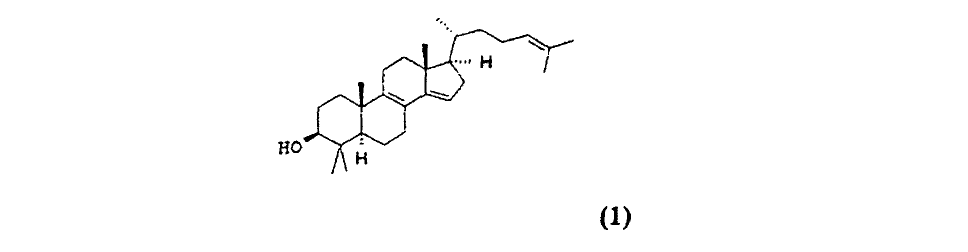 from compounds of the general formula 6 according to Claim 1 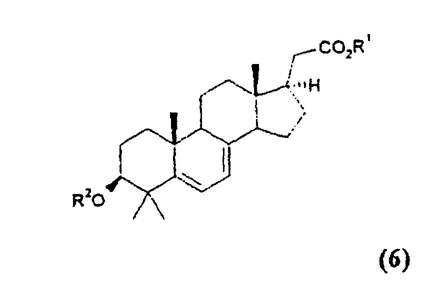 in which
R¹ and R² have the meanings indicated in step (c) of Claim 1,
j) by alkylation to give the compounds of the general formula 12 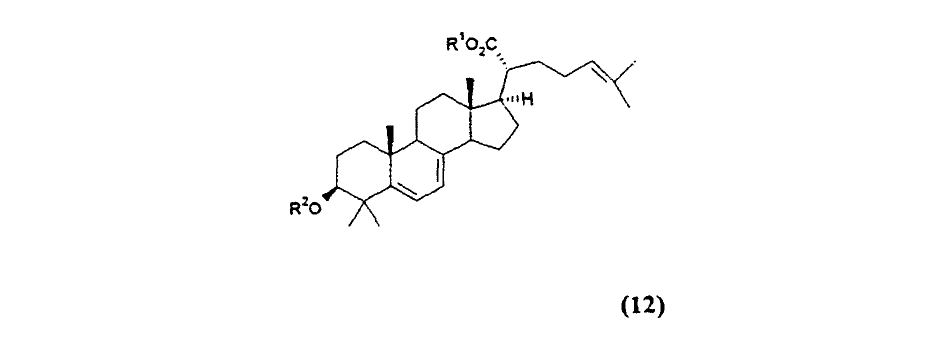 in which
R¹ and R² have the abovementioned meanings,
k) reduction of the ester group to the hydroxymethyl compound of the general formula 13 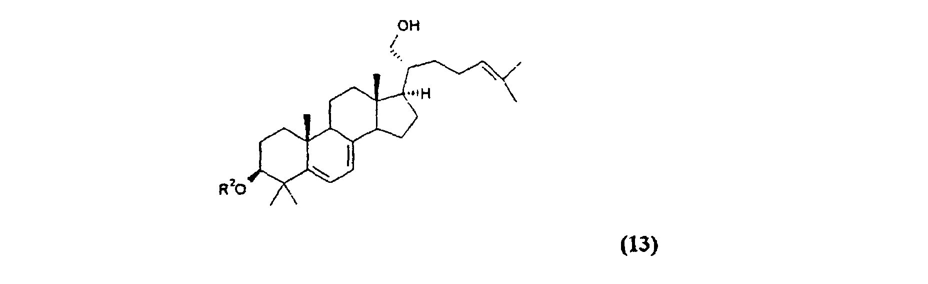 in which
R² has the abovementioned meaning,
l) by conversion of the primary hydroxyl group to a leaving group in compounds of the general formula 14 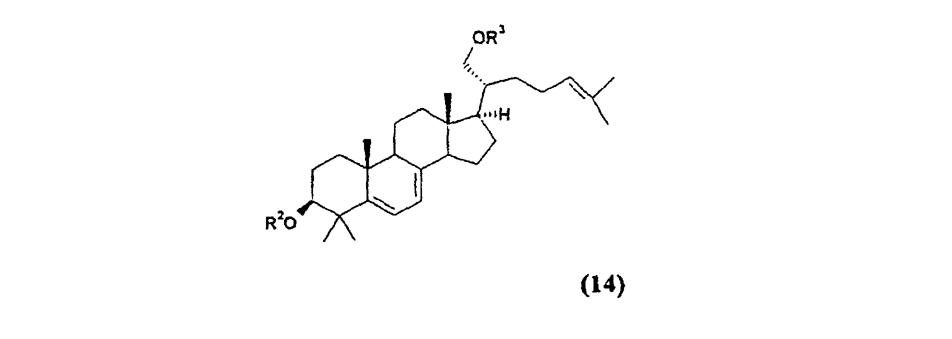 in which
R² has the abovementioned meanings and R³ has the meanings mentioned in Claim 1,
m) by reductive removal of the -OR₃ group in compounds of the general formula 15 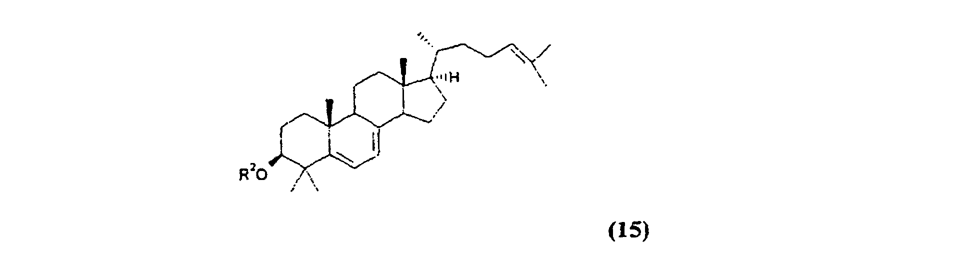 in which
R² has the abovementioned meaning,
n) by isomerization in compounds of the general formula 11 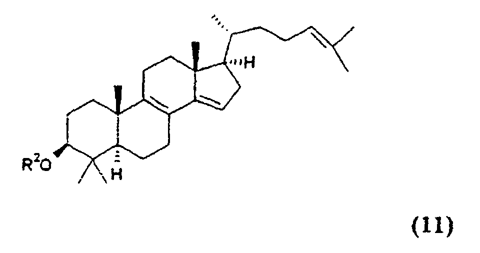 in which
R² has the abovementioned meaning or is hydrogen, and removal of the protective group if R² is a protective group.

3. Compounds of the general formula 3 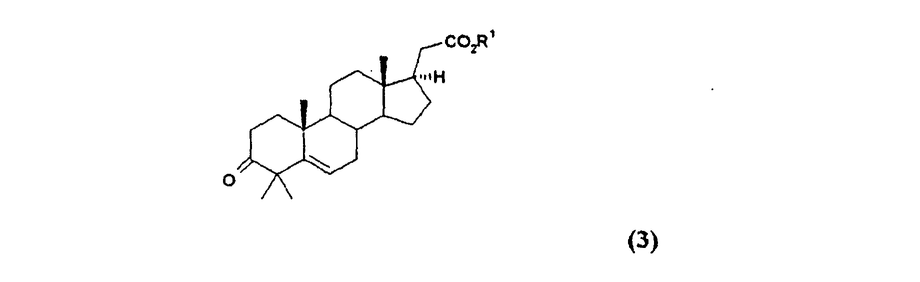 in which
R¹ is hydrogen, branched or unbranched C₁-C₆ alkyl, phenyl, benzyl, ortho-, meta- or para-methylphenyl.

4. Compounds of the general formula 4 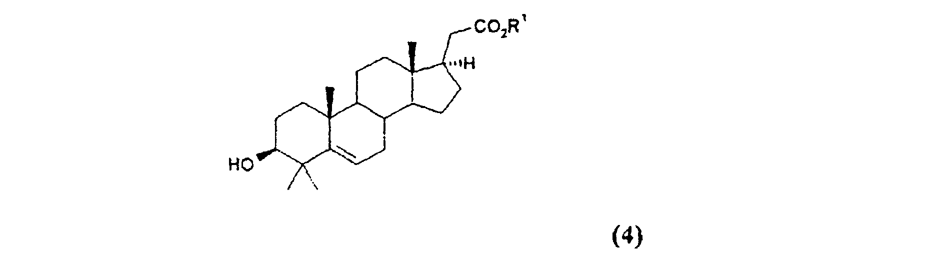 in which
R¹ is hydrogen, branched or unbranched C₁-C₆ alkyl, phenyl, benzyl, ortho-, meta- or para-methylphenyl.

5. Compounds of the general formula 5 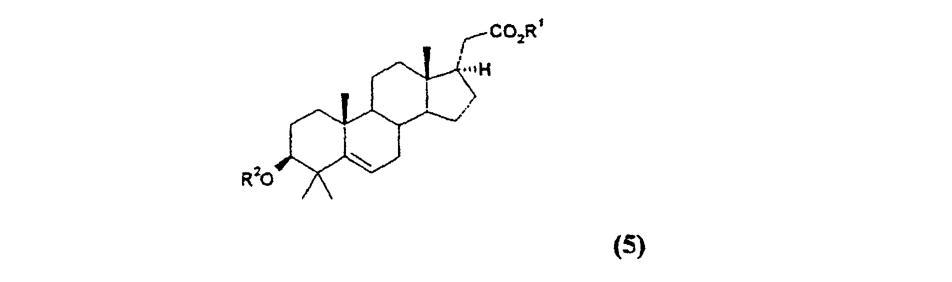 in which
R¹ is branched or unbranched C₁-C₆ alkyl, phenyl, benzyl, ortho-, meta- or para-methylphenyl and
R² is hydrogen, esters of aliphatic and aromatic carboxylic acids, or silyl ethers.

6. Compounds of the general formula 6 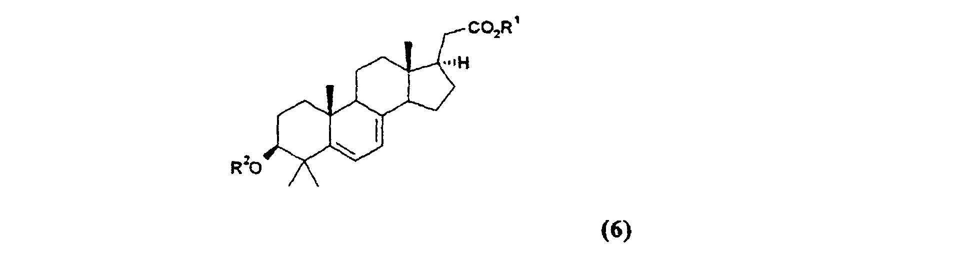 in which
R¹ is branched or unbranched C₁-C₆ alkyl, phenyl, benzyl, ortho-, meta- or para-methylphenyl and
R² is hydrogen, esters of aliphatic and aromatic carboxylic acids, or silyl ethers.

7. Compounds of the general formula 7 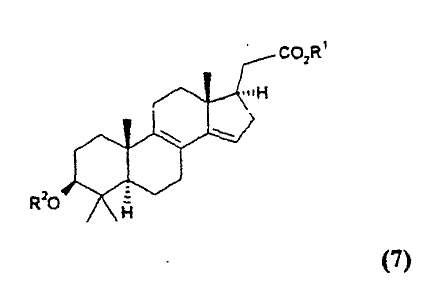 in which
R¹ is hydrogen, branched or unbranched C₁-C₆ alkyl, phenyl, benzyl, ortho-, meta- or para-methylphenyl and
R² is hydrogen, esters of aliphatic and aromatic carboxylic acids, or silyl ethers.

8. Compounds of the general formula 8 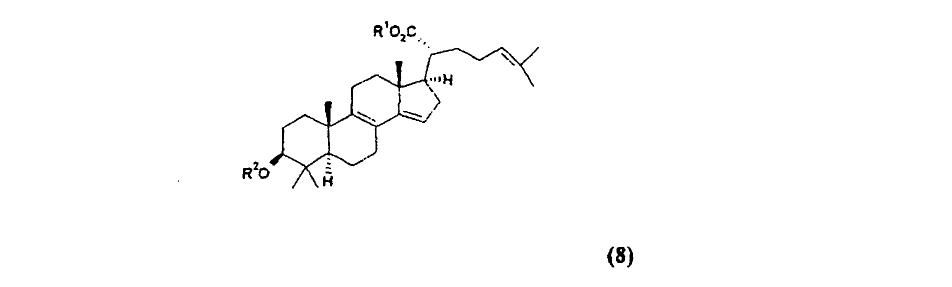 in which
R¹ is hydrogen, branched or unbranched C₁-C₆ alkyl, phenyl, benzyl, ortho-, meta- or para-methylphenyl and
R² is hydrogen, esters of aliphatic and aromatic carboxylic acids, acetal protective groups or silyl ethers.

9. Compounds of the general formula 9 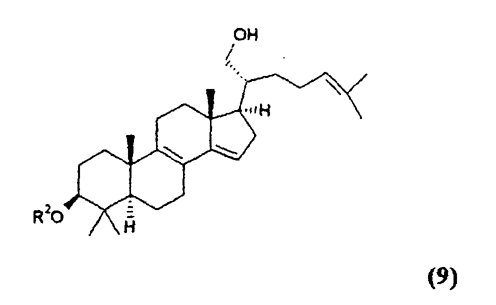 in which
R² is hydrogen, esters of aliphatic and aromatic carboxylic acids, acetal protective groups or silyl ethers.

10. Compounds of the general formula 10 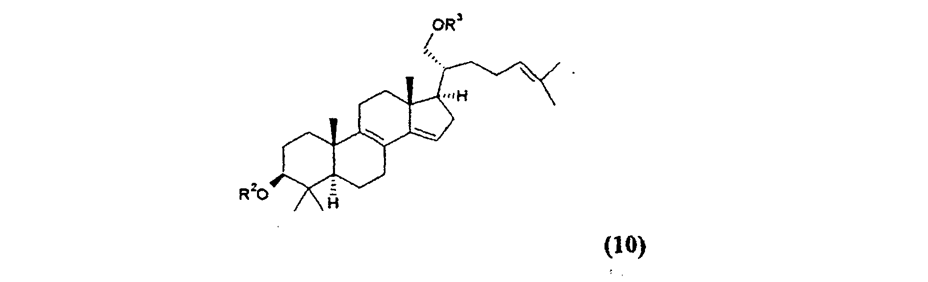 in which
R² is hydrogen, esters of aliphatic and aromatic carboxylic acids, acetal protective groups or silyl ethers and
R³ is SO₂R⁴, where R⁴ is branched or unbranched C₁-C₆ alkyl, phenyl, benzyl, ortho-, meta- or para-methylphenyl or 2,4,6-trimethylphenyl.

11. Compounds of the general formula 12 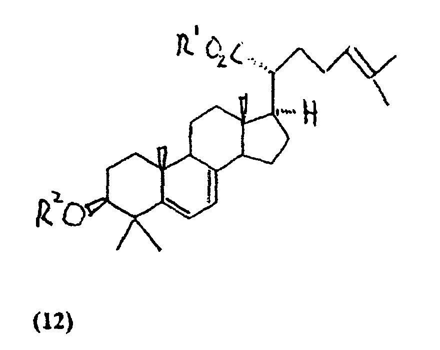 in which
R¹ is branched or unbranched C₁-C₆ alkyl, phenyl, benzyl, ortho-, meta- or para-methylphenyl and
R² is hydrogen, esters of aliphatic and aromatic carboxylic acids, acetal protective groups or silyl ethers.

12. Hydroxymethyl compound of the general formula 13 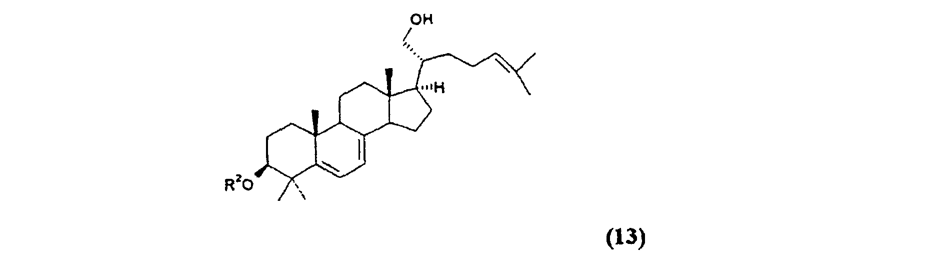 in which
R² is esters of aliphatic and aromatic carboxylic acids, acetal protective groups or silyl ethers.

13. Compounds of the general formula 14 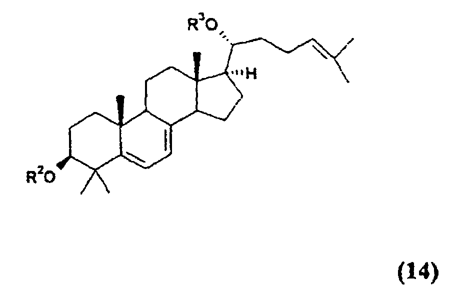 in which
R² is esters of aliphatic and aromatic carboxylic acids, acetal protective groups or silyl ethers and
R³ is SO₂R⁴, where R⁴ is branched or unbranched C₁-C₆ alkyl, phenyl, benzyl, ortho-, meta- or para-methylphenyl or 2,4,6-trimethylphenyl.

14. Compounds of the general formula 15 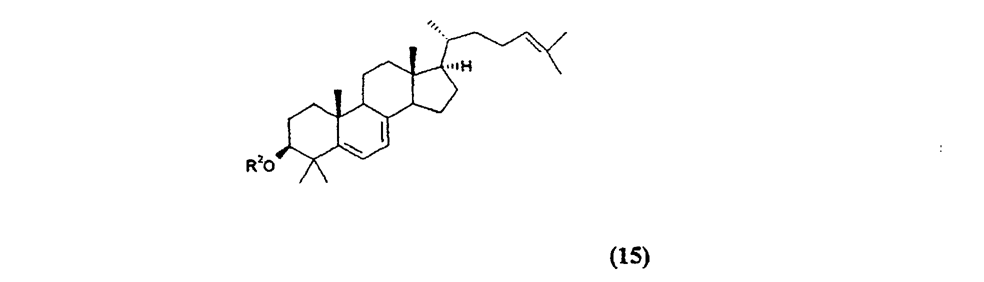 in which
R² is esters of aliphatic and aromatic carboxylic acids, acetal protective groups or silyl ethers.

## Revendications

1. Procédé de préparation du 4,4-diméthyl-5α-cholesta-8,14,24-trién-3β-ol de formule 1 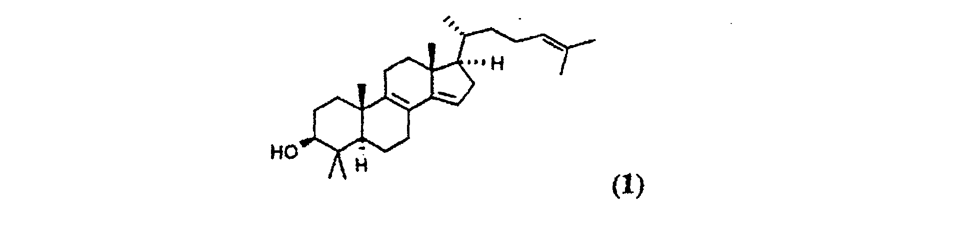 à partir de dérivés esters de l'acide 3-oxoprégn-4-én-21-oïque de formule générale 2 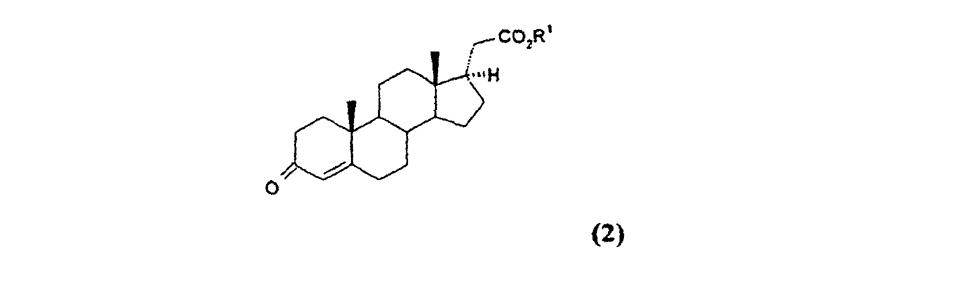 dans laquelle
R¹ représente un hydrogène, un alkyle en C₁-C₆ ramifié ou non ramifié, un phényle, un benzyle, un ortho-, un méta- ou un para-méthylphényle,
a) par réaction en présence de bases et d'un agent de méthylation pour donner lieu à des composés de formule générale 3 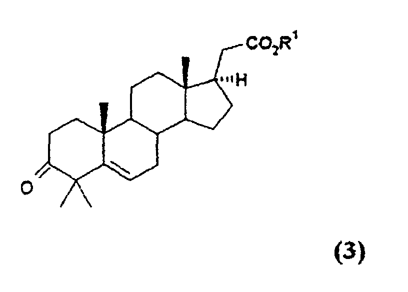 dans laquelle
R¹ présente la signification susmentionnée,
b) par réduction en composés de formule générale 4 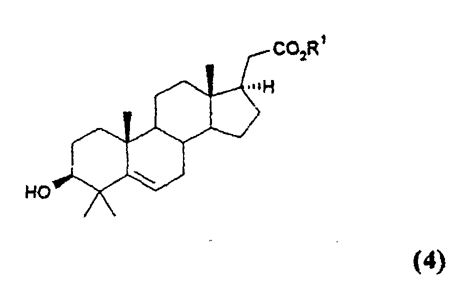 dans laquelle
R¹ présente la signification susmentionnée,
c) par transformation en un groupe hydroxy-3-protégé des composés de formule générale 5 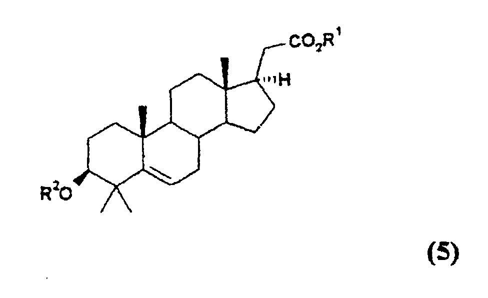 dans laquelle
R¹ présente la signification susmentionnée et
R² représente un ester d'acides carboxyliques aliphatiques et aromatiques, un groupe protecteur acétal, un groupe protecteur éther ou un éther silylique,
d) par déshydrogénation en composés de formule générale 6 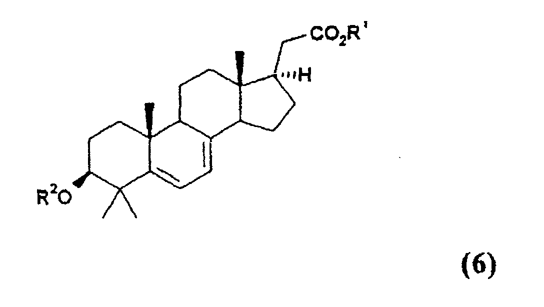 dans laquelle
R¹ et R² présentent les significations susmentionnées,
e) par isomérisation en composés de formule générale 7 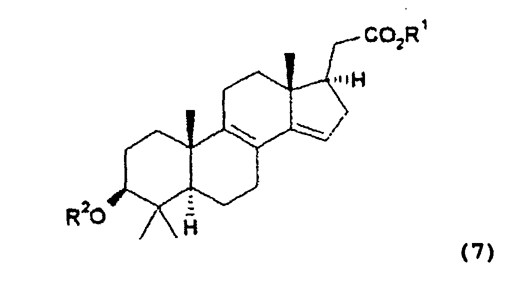 dans laquelle
R¹ et R² présentent les significations susmentionnées, (e') le groupe protecteur souhaité étant éventuellement introduit avant l'étape d'alkylation (f),
f) par alkylation en composés de formule générale 8 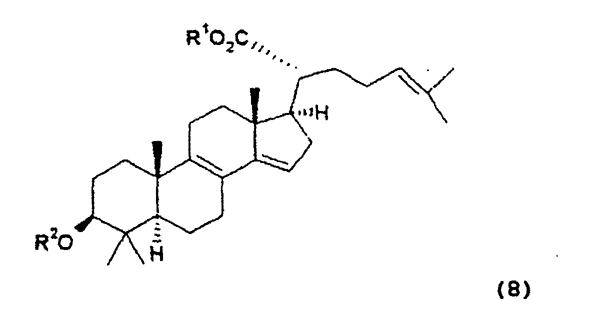 dans laquelle
R¹ et R² présentent les significations susmentionnées où R² peut également représenter un hydrogène,
g) par réduction de l'ester en un groupe hydroxyméthyle des composés de formule générale 9 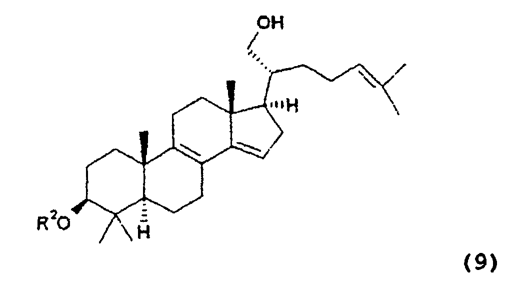 dans laquelle
R² présente la signification susmentionnée ou représente un hydrogène,
h) par transformation du groupe hydroxy primaire en un groupe partant dans des composés de formule générale 10 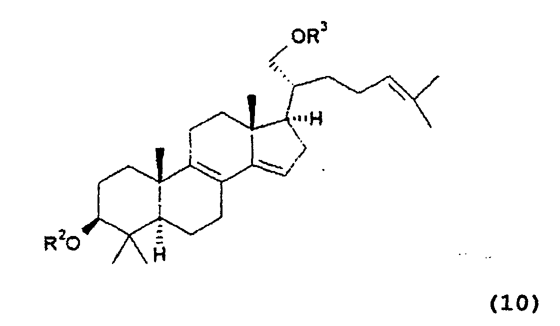 dans laquelle
R² présente la signification susmentionnée ou représente un hydrogène et
R³ représente le radical SO₂R⁴, dans lequel R⁴ représente un alkyle en C₁-C₆ ramifié ou non ramifié, un phényle, un benzyle, un ortho-, un méta- ou un para-méthylphényle ou un 2,4,6-triméthylphényle, et
i) par élimination réductive du groupement -OR³- dans des composés de formule générale 11 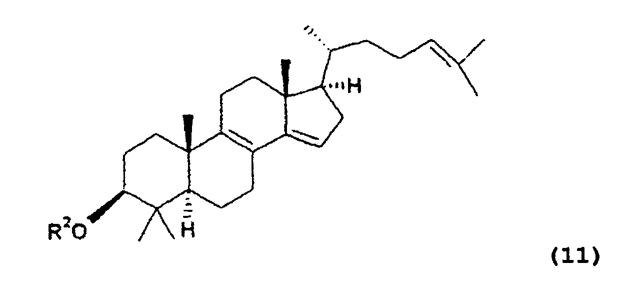 dans laquelle
R² présente la signification susmentionnée ou représente un hydrogène,
et par élimination du groupe protecteur, si R² représente un groupe protecteur.

2. Procédé de préparation du 4,4-diméthyl-5α-cholesta-8,14,24-trién-3β-ol de formule 1 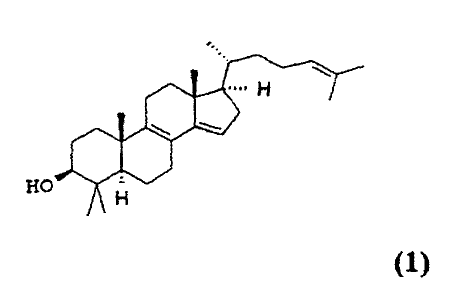 à partir de composés de formule générale 6 selon la revendication 1 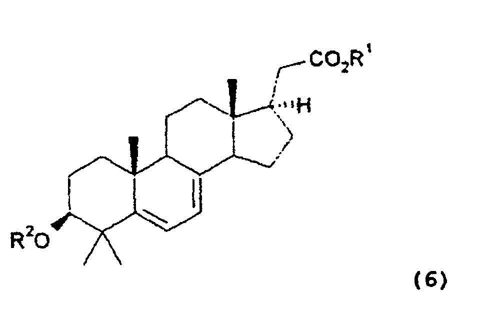 dans laquelle
R¹ et R² présentent les significations indiquées dans l'étape (c) de la revendication 1,
j) par alkylation en composés de formule générale 12 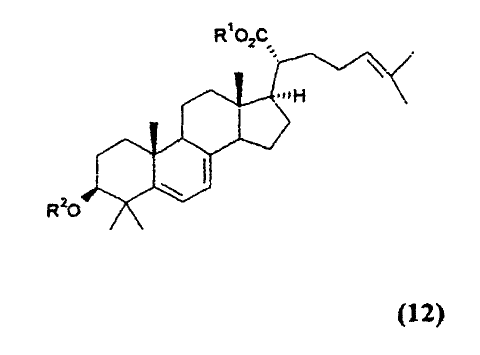 dans laquelle
R¹ et R² présentent les significations susmentionnées,
k) par réduction du groupe ester en un composé hydroxyméthyle de formule générale 13 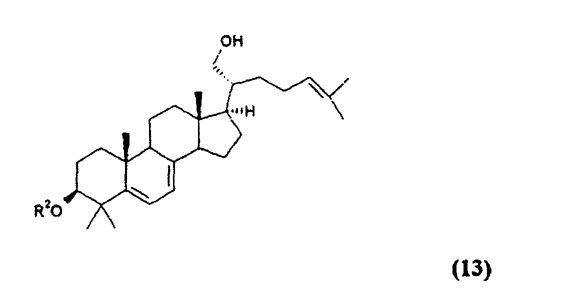 dans laquelle
R² présente la signification susmentionnée,
l) par transformation du groupe hydroxy primaire en un groupe partant dans des composés de formule générale 14 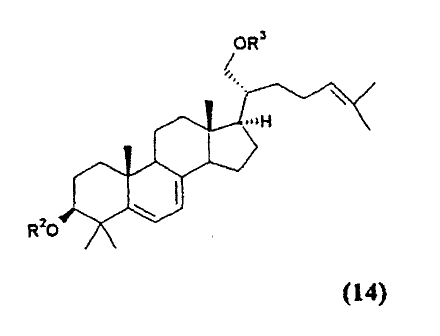 dans laquelle
R² présente la signification susmentionnée et R³ présente les significations mentionnées dans la revendication 1,
m) par élimination réductive du groupement -OR³- dans des composés de formule générale 15 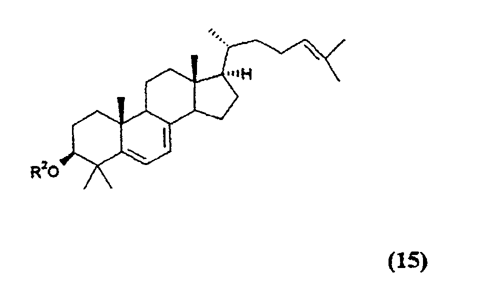 dans laquelle
R² présente la signification susmentionnée,
n) par isomérisation en composés de formule générale 11 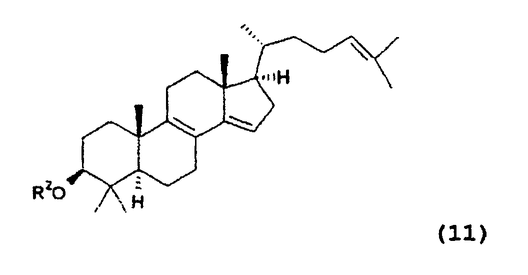 dans laquelle
R² présente la signification susmentionnée ou représente un hydrogène, et par élimination du groupe protecteur, si R² représente un groupe protecteur.

3. Composés de formule générale 3 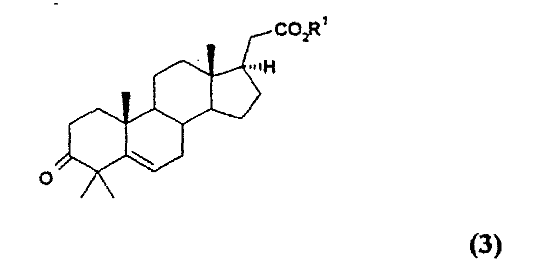 dans laquelle
R¹ représente un hydrogène, un alkyle en C₁-C₆ ramifié ou non ramifié, un phényle, un benzyle, un ortho-, un méta- ou un para-méthylphényle.

4. Composés de formule générale 4 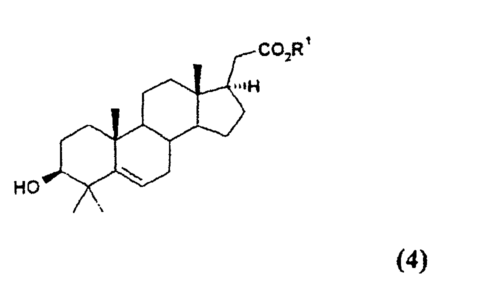 dans laquelle
R¹ représente un hydrogène, un alkyle en C₁-C₆ ramifié ou non ramifié, un phényle, un benzyle, un ortho-, un méta- ou un para-méthylphényle.

5. Composés de formule générale 5 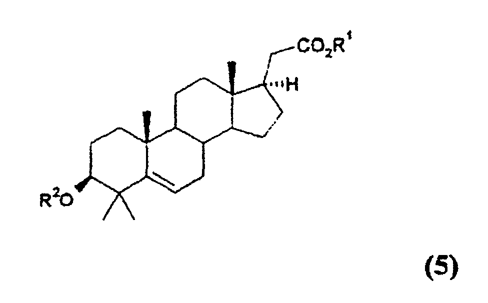 dans laquelle
R¹ représente un alkyle en C₁-C₆ ramifié ou non ramifié, un phényle, un benzyle, un ortho-, un méta- ou un para-méthylphényle et
R² représente un hydrogène, un ester d'acides carboxyliques aliphatiques et aromatiques ou un éther silylique.

6. Composés de formule générale 6 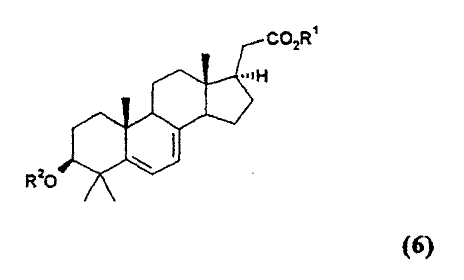 dans laquelle
R¹ représente un alkyle en C₁-C₆ ramifié ou non ramifié, un phényle, un benzyle, un ortho-, un méta- ou un para-méthylphényle et
R² représente un hydrogène, un ester d'acides carboxyliques aliphatiques et aromatiques ou un éther silylique.

7. Composés de formule générale 7 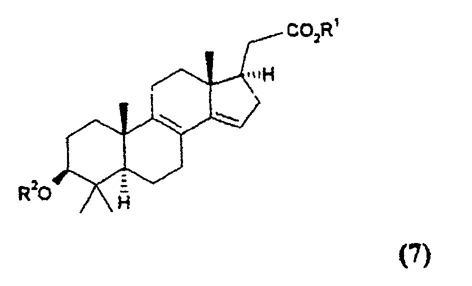 dans laquelle
R¹ représente un hydrogène, un alkyle en C₁-C₆ ramifié ou non ramifié, un phényle, un benzyle, un ortho-, un méta- ou un para-méthylphényle et
R² représente un hydrogène, un ester d'acides carboxyliques aliphatiques et aromatiques ou un éther silylique.

8. Composés de formule générale 8 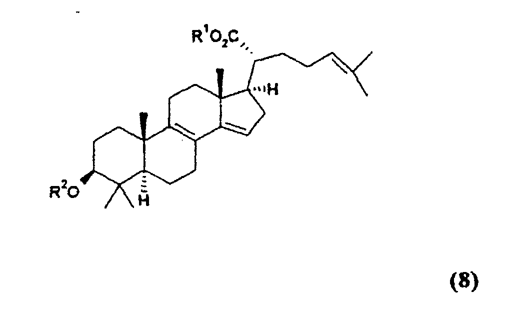 dans laquelle
R¹ représente un hydrogène, un alkyle en C₁-C₆ ramifié ou non ramifié, un phényle, un benzyle, un ortho-, un méta- ou un para-méthylphényle et
R² représente un hydrogène, un ester d'acides carboxyliques aliphatiques et aromatiques, un groupe protecteur acétal ou un éther silylique.

9. Composés de formule générale 9 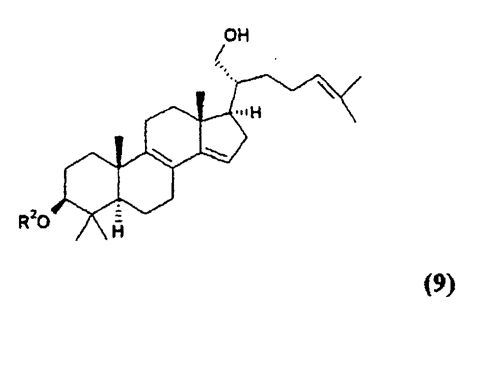 dans laquelle
R² représente un hydrogène, un ester d'acides carboxyliques aliphatiques et aromatiques, un groupe protecteur acétal ou un éther silylique.

10. Composés de formule générale 10 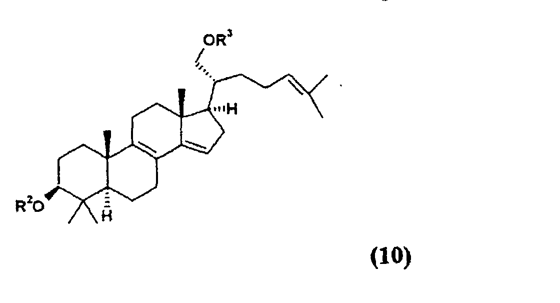 dans laquelle
R² représente un hydrogène, un ester d'acides carboxyliques aliphatiques et aromatiques, un groupe protecteur acétal ou un éther silylique et
R³ représente SO₂R⁴ où R⁴ représente un alkyle en C₁-C₆ ramifié ou non ramifié, un phényle, un benzyle, un ortho-, un méta- ou un para-méthylphényle ou un 2,4,6-triméthylphényle.

11. Composés de formule générale 12 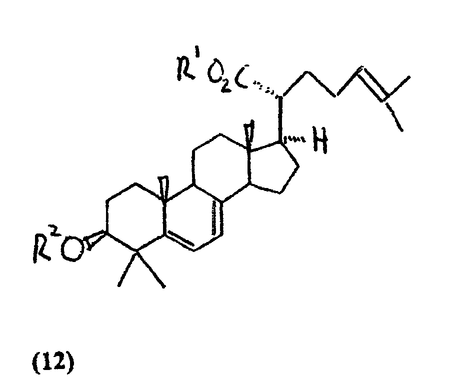 dans laquelle
R¹ représente un alkyle en C₁-C₆ ramifié ou non ramifié, un phényle, un benzyle, un ortho-, un méta- ou un para-méthylphényle et
R² représente un hydrogène, un ester d'acides carboxyliques aliphatiques et aromatiques, un groupe protecteur acétal ou un éther silylique.

12. Composé hydroxyméthyle de formule générale 13 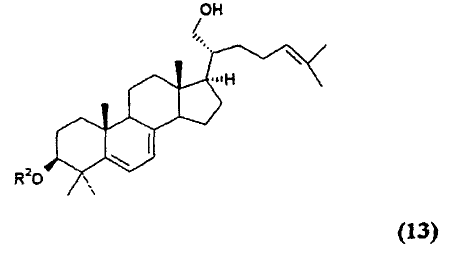 dans laquelle
R² représente un ester d'acides carboxyliques aliphatiques et aromatiques, un groupe protecteur acétal ou un éther silylique.

13. Composés de formule générale 14 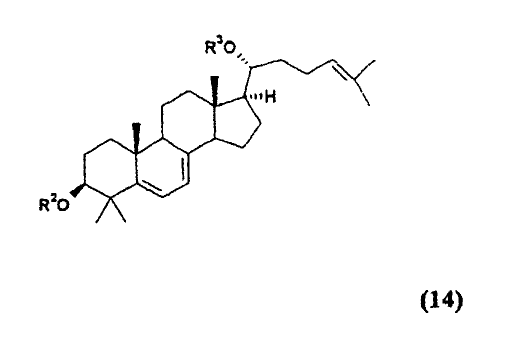 dans laquelle
R² représente un ester d'acides carboxyliques aliphatiques et aromatiques, un groupe protecteur acétal ou un éther silylique,
R³ représente SO₂R⁴ où R⁴ représente un alkyle en C₁-C₆ ramifié ou non ramifié, un phényle, un benzyle, un ortho-, un méta- ou un para-méthylphényle ou un 2,4,6-triméthylphényle.

14. Composés de formule générale 15 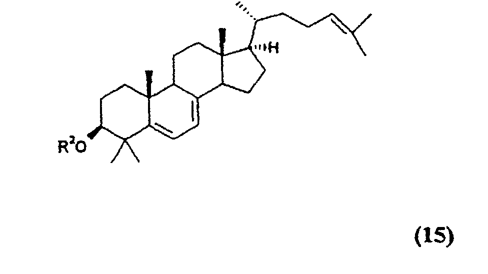 dans laquelle
R² représente un ester d'acides carboxyliques aliphatiques et aromatiques, un groupe protecteur acétal ou un éther silylique.
